# EUROPEAN PATENT SPECIFICATION

(11) **EP 2 252 692 B1**
(45) Date of publication and mention of the grant of the patent: **27.11.2013**
(21) Application number: 09719164.7
(22) Date of filing: 13.03.2009
(51) Int. Cl.: C12N 15/54, C12N 9/12, C12N 15/63, C12N 1/21, C12Q 1/68

(54) **A THERMOSTABLE DNA POLYMERASE FROM PALAEOCOCCUS FERROPHILUS**
THERMOSTABILE DNA POLYMERASE VON PALAEOCOCCUS FERROPHILUS
ADN POLYMÉRASE THERMOSTABLE DE PALAEOCOCCUS FERROPHILUS& xA;

(30) Priority: 14.03.2008 GB 0804722; 14.03.2008 US 69397 P
(43) Date of publication of application: 24.11.2010
(73) Proprietor: Genesys Ltd, Surrey GU16 7PL (GB)
(72) Inventor: CLARK, Duncan, Camberley Surrey GU16 7PL (GB); MORANT, Nicholas, Camberley Surrey GU16 7PL (GB)
(74) Representative: Turner, Rhiannon Rosalind
(86) International application number: PCT/GB2009/050246
(87) International publication number: WO 2009/112867

(56) References cited:
- DATABASE Geneseq [Online] 23 February 2006 (2006-02-23), "Thermococcus sp. DNA polymerase SEQ ID NO 32." XP002534619 retrieved from EBI accession no. GSP:AEE85221 Database accession no. AEE85221 -& EP 1 757 698 A (TAKARA BIO INC [JP]) 28 February 2007 (2007-02-28) -& WO 2005/118815 A (TAKARA BIO INC [JP]; SATO YOSHIMI [JP]; NISHIWAKI KAZUE [JP]; SHIMADA) 15 December 2005 (2005-12-15)
- DATABASE UniProt [Online] 24 January 2006 (2006-01-24), "RecName: Full=DNA polymerase; EC=<A HREF="http://srs.ebi.ac.uk/srsbin/cgi-bin/ wgetz?[enzyme-ECNumber:2.7.7.7]+-e">2.7.7. 7</A>;" XP002534622 retrieved from EBI accession no. UNIPROT:Q2Q453 Database accession no. Q2Q453
- DATABASE UniProt [Online] 9 January 2007 (2007-01-09), "RecName: Full=DNA polymerase; EC=<A HREF="http://srs.ebi.ac.uk/srsbin/cgi-bin/ wgetz?[enzyme-ECNumber:2.7.7.7]+-e">2.7.7. 7</A>;" XP002534623 retrieved from EBI accession no. UNIPROT:A0SXL5 Database accession no. A0SXL5
- TAKAI K ET AL: "Palaeococcus ferrophilus gen. nov., sp. nov., a barophilic, hyperthermophilic archaeon from a deep-sea hydrothermal vent chimney." INTERNATIONAL JOURNAL OF SYSTEMATIC AND EVOLUTIONARY MICROBIOLOGY MAR 2000, vol. 50 Pt 2, March 2000 (2000-03), pages 489-500, XP002534638 ISSN: 1466-5026

### SEQUENCE LISTING

<110> GeneSys Ltd Clark, Duncan R Morant, Nicholas
<120> Enzyme
<130> P1443EP00
<140> 09719164.7
   <141> 2009-03-13
<150> GB0804722.7
   <151> 2008-03-14
<150> US 61/069397
   <151> 2008-03-14
<160> 52
<170> PatentIn version 3.5
<210> 1
   <211> 775
   <212> PRT
   <213> Palaeococcus ferrophilus
<400> 1
   Met Ile Leu Asp Ala Asp Tyr Ile Thr Glu Asn Gly Lys Pro Val Val
   1               5                   10                  15
   Arg Ile Phe Lys Lys Glu Asn Gly Glu Phe Lys Val Glu Tyr Asp Arg
               20                  25                  30
   Asn Phe Glu Pro Tyr Ile Tyr Ala Leu Leu Lys Asp Asp Ser Ala Ile
           35                  40                  45
   Glu Glu Ile Lys Lys Ile Thr Ala Glu Arg His Gly Thr Val Val Arg
       50                  55                  60
   Ile Thr Lys Ala Glu Lys Val Glu Arg Lys Phe Leu Gly Arg Pro Val
   65                  70                  75                  80
   Glu Val Trp Lys Leu Tyr Phe Thr His Pro Gln Asp Val Pro Ala Ile
                   85                  90                  95
   Arg Asp Lys Ile Arg Ser His Pro Ala Val Val Asp Ile Tyr Glu Tyr
               100                 105                 110
   Asp Ile Pro Phe Ala Lys Arg Tyr Leu Ile Asp Lys Gly Leu Val Pro
           115                 120                 125
   Met Glu Gly Asp Glu Glu Leu Lys Met Leu Ala Phe Asp Ile Glu Thr
       130                 135                 140
   Leu Tyr His Glu Gly Glu Glu Phe Ala Glu Gly Pro Ile Leu Met Ile
   145                 150                 155                 160
   Ser Tyr Ala Asp Glu Ser Glu Ala Arg Val Ile Thr Trp Lys Lys Val
                   165                 170                 175
   Asp Leu Pro Tyr Val Asp Ala Val Ser Thr Glu Lys Asp Met Ile Lys
               180                 185                 190
   Ala Phe Leu Arg Val Val Lys Glu Lys Asp Pro Asp Val Leu Ile Thr
           195                 200                 205
   Tyr Asn Gly Asp Asn Phe Asp Phe Ala Tyr Leu Lys Lys Arg Cys Glu
       210                 215                 220
   Lys Leu Gly Val Lys Phe Ile Leu Gly Arg Asp Gly Ser Glu Pro Lys
   225                 230                 235                 240
   Ile Gln Arg Met Gly Asp Arg Phe Ala Val Asp Val Lys Gly Arg Ile
                   245                 250                 255
   His Phe Asp Leu Tyr Pro Val Ile Arg Arg Thr Ile Asn Leu Pro Thr
               260                 265                 270
   Tyr Thr Leu Glu Ala Val Tyr Glu Ala Ile Phe Gly Arg Pro Lys Glu
           275                 280                 285
   Lys Val Tyr Ala Glu Glu Ile Ala Gln Ala Trp Glu Thr Asn Glu Gly
       290                 295                 300
   Leu Glu Arg Val Ala Arg Tyr Ser Met Glu Asp Ala Lys Val Thr Tyr
   305                 310                 315                 320
   Glu Leu Gly Lys Glu Phe Phe Pro Met Glu Ala Gln Leu Ser Arg Leu
                   325                 330                 335
   Ile Gly Gln Pro Leu Trp Asp Val Ser Arg Ser Ser Thr Gly Asn Leu
               340                 345                 350
   Val Glu Trp Phe Leu Leu Arg Lys Ala Tyr Glu Arg Asn Glu Leu Ala
           355                 360                 365
   Pro Asn Lys Pro Ser Gly Arg Glu Tyr Asp Glu Arg Arg Gly Gly Tyr
       370                 375                 380
   Ala Gly Gly Tyr Val Lys Glu Pro Glu Lys Gly Leu Trp Glu Asn Ile
   385                 390                 395                 400
   Val Tyr Leu Asp Tyr Lys Ser Leu Tyr Pro Ser Ile Ile Ile Thr His
                   405                 410                 415
   Asn Val Ser Pro Asp Thr Leu Asn Arg Glu Gly Cys Lys Glu Tyr Asp
               420                 425                 430
   Val Ala Pro Gln Val Gly His Arg Phe Cys Lys Asp Phe Pro Gly Phe
           435                 440                 445
   Ile Pro Ser Leu Leu Gly Asp Leu Leu Glu Glu Arg Gln Lys Ile Lys
       450                 455                 460
   Arg Lys Met Lys Ala Thr Ile Asp Pro Ile Glu Arg Arg Leu Leu Asp
   465                 470                 475                 480
   Tyr Arg Gln Arg Ala Ile Lys Ile Leu Ala Asn Ser Tyr Tyr Gly Tyr
                   485                 490                 495
   Tyr Gly Tyr Ala Arg Ala Arg Trp Tyr Cys Lys Glu Cys Ala Glu Ser
               500                 505                 510
   Val Thr Ala Trp Gly Arg Glu Tyr Ile Glu Met Ser Ile Arg Glu Ile
           515                 520                 525
   Glu Glu Lys Tyr Gly Phe Lys Val Leu Tyr Ala Asp Thr Asp Gly Phe
       530                 535                 540
   His Ala Thr Ile Pro Gly Glu Asp Ala Glu Thr Ile Lys Lys Lys Ala
   545                 550                 555                 560
   Met Glu Phe Leu Lys Tyr Ile Asn Ser Lys Leu Pro Gly Ala Leu Glu
                   565                 570                 575
   Leu Glu Tyr Glu Gly Phe Tyr Arg Arg Gly Phe Phe Val Thr Lys Lys
               580                 585                 590
   Lys Tyr Ala Val Ile Asp Glu Glu Gly Lys Ile Thr Thr Arg Gly Leu
           595                 600                 605
   Glu Ile Val Arg Arg Asp Trp Ser Glu Ile Ala Lys Glu Thr Gln Ala
       610                 615                 620
   Arg Val Leu Glu Ala Leu Leu Lys Asp Gly Asn Val Glu Glu Ala Val
   625                 630                 635                 640
   Ser Ile Val Lys Glu Val Thr Glu Lys Leu Ser Lys Tyr Glu Val Pro
                   645                 650                 655
   Pro Glu Lys Leu Val Ile His Glu Gln Ile Thr Arg Glu Leu Lys Asp
               660                 665                 670
   Tyr Lys Ala Thr Gly Pro His Val Ala Ile Ala Lys Arg Leu Ala Ala
           675                 680                 685
   Arg Gly Val Lys Ile Arg Pro Gly Thr Val Ile Ser Tyr Ile Val Leu
       690                 695                 700
   Lys Gly Ser Gly Arg Ile Gly Asp Arg Ala Ile Pro Phe Asp Glu Phe
   705                 710                 715                 720
   Asp Pro Ala Lys His Arg Tyr Asp Ala Glu Tyr Tyr Ile Glu Asn Gln
                   725                 730                 735
   Val Leu Pro Ala Val Glu Arg Ile Leu Lys Ala Phe Gly Tyr Arg Lys
               740                 745                 750
   Glu Asp Leu Arg Tyr Gln Lys Thr Arg Gln Val Gly Leu Gly Ala Trp
           755                 760                 765
   Leu Lys Pro Lys Gly Lys Lys
       770                 775
<210> 2
   <211> 1305
   <212> PRT
   <213> Palaeococcus ferrophilus
<400> 2
   Met Ile Leu Asp Ala Asp Tyr Ile Thr Glu Asn Gly Lys Pro Val Val
   1               5                   10                  15
   Arg Ile Phe Lys Lys Glu Asn Gly Glu Phe Lys Val Glu Tyr Asp Arg
               20                  25                  30
   Asn Phe Glu Pro Tyr Ile Tyr Ala Leu Leu Lys Asp Asp Ser Ala Ile
           35                  40                  45
   Glu Glu Ile Lys Lys Ile Thr Ala Glu Arg His Gly Thr Val Val Arg
       50                  55                  60
   Ile Thr Lys Ala Glu Lys Val Glu Arg Lys Phe Leu Gly Arg Pro Val
   65                  70                  75                  80
   Glu Val Trp Lys Leu Tyr Phe Thr His Pro Gln Asp Val Pro Ala Ile
                   85                  90                  95
   Arg Asp Lys Ile Arg Ser His Pro Ala Val Val Asp Ile Tyr Glu Tyr
               100                 105                 110
   Asp Ile Pro Phe Ala Lys Arg Tyr Leu Ile Asp Lys Gly Leu Val Pro
           115                 120                 125
   Met Glu Gly Asp Glu Glu Leu Lys Met Leu Ala Phe Asp Ile Glu Thr
       130                 135                 140
   Leu Tyr His Glu Gly Glu Glu Phe Ala Glu Gly Pro Ile Leu Met Ile
   145                 150                 155                 160
   Ser Tyr Ala Asp Glu Ser Glu Ala Arg Val Ile Thr Trp Lys Lys Val
                   165                 170                 175
   Asp Leu Pro Tyr Val Asp Ala Val Ser Thr Glu Lys Asp Met Ile Lys
               180                 185                 190
   Ala Phe Leu Arg Val Val Lys Glu Lys Asp Pro Asp Val Leu Ile Thr
           195                 200                 205
   Tyr Asn Gly Asp Asn Phe Asp Phe Ala Tyr Leu Lys Lys Arg Cys Glu
       210                 215                 220
   Lys Leu Gly Val Lys Phe Ile Leu Gly Arg Asp Gly Ser Glu Pro Lys
   225                 230                 235                 240
   Ile Gln Arg Met Gly Asp Arg Phe Ala Val Asp Val Lys Gly Arg Ile
                   245                 250                 255
   His Phe Asp Leu Tyr Pro Val Ile Arg Arg Thr Ile Asn Leu Pro Thr
               260                 265                 270
   Tyr Thr Leu Glu Ala Val Tyr Glu Ala Ile Phe Gly Arg Pro Lys Glu
           275                 280                 285
   Lys Val Tyr Ala Glu Glu Ile Ala Gln Ala Trp Glu Thr Asn Glu Gly
       290                 295                 300
   Leu Glu Arg Val Ala Arg Tyr Ser Met Glu Asp Ala Lys Val Thr Tyr
   305                 310                 315                 320
   Glu Leu Gly Lys Glu Phe Phe Pro Met Glu Ala Gln Leu Ser Arg Leu
                   325                 330                 335
   Ile Gly Gln Pro Leu Trp Asp Val Ser Arg Ser Ser Thr Gly Asn Leu
               340                 345                 350
   Val Glu Trp Phe Leu Leu Arg Lys Ala Tyr Glu Arg Asn Glu Leu Ala
           355                 360                 365
   Pro Asn Lys Pro Ser Gly Arg Glu Tyr Asp Glu Arg Arg Gly Gly Tyr
       370                 375                 380
   Ala Gly Gly Tyr Val Lys Glu Pro Glu Lys Gly Leu Trp Glu Asn Ile
   385                 390                 395                 400
   Val Tyr Leu Asp Tyr Lys Ser Leu Tyr Pro Ser Ile Ile Ile Thr His
                   405                 410                 415
   Asn Val Ser Pro Asp Thr Leu Asn Arg Glu Gly Cys Lys Glu Tyr Asp
               420                 425                 430
   Val Ala Pro Gln Val Gly His Arg Phe Cys Lys Asp Phe Pro Gly Phe
           435                 440                 445
   Ile Pro Ser Leu Leu Gly Asp Leu Leu Glu Glu Arg Gln Lys Ile Lys
       450                 455                 460
   Arg Lys Met Lys Ala Thr Ile Asp Pro Ile Glu Arg Arg Leu Leu Asp
   465                 470                 475                 480
   Tyr Arg Gln Arg Ala Ile Lys Ile Leu Ala Asn Ser Ile Leu Pro Asp
                   485                 490                 495
   Glu Trp Leu Pro Ile Ile Glu Asn Gly Thr Val Arg Phe Val Arg Ile
               500                 505                 510
   Gly Glu Phe Ile Asp Trp Lys Met Asp Glu Asn Ala Glu Arg Val His
           515                 520                 525
   Arg Glu Gly Glu Thr Glu Ile Leu Glu Val Ser Gly Leu Glu Val Gln
       530                 535                 540
   Ser Phe Asn Arg Glu Thr Lys Lys Ala Glu Leu Lys Arg Val Lys Ala
   545                 550                 555                 560
   Leu Ile Arg His Arg Tyr Ser Gly Lys Ala Tyr Asn Ile Lys Leu Lys
                   565                 570                 575
   Ser Gly Arg Arg Ile Lys Ile Thr Ser Gly His Ser Leu Phe Val Glu
               580                 585                 590
   Val Thr Gly Asp Glu Leu Lys Pro Gly Asp Leu Val Ala Val Pro Arg
           595                 600                 605
   Arg Val Lys Leu Pro Glu Arg Asn His Val Leu Asn Leu Val Glu Leu
       610                 615                 620
   Leu Leu Gly Phe Pro Glu Asp Glu Thr Ser Asp Ile Val Met Thr Ile
   625                 630                 635                 640
   Pro Val Lys Glu Arg Lys Asn Phe Phe Lys Gly Met Leu Arg Thr Leu
                   645                 650                 655
   Arg Trp Ile Phe Gly Glu Glu Lys Arg Pro Arg Thr Ala Arg Arg Tyr
               660                 665                 670
   Leu Lys His Leu Glu Asp Leu Gly Tyr Val Arg Leu Lys Lys Ile Gly
           675                 680                 685
   Tyr Glu Val Leu Asp Trp Glu Ala Leu Arg Lys Tyr Arg Arg Leu Tyr
       690                 695                 700
   Glu Ala Leu Val Glu Lys Ile Arg Tyr Asn Gly Asn Lys Arg Glu Tyr
   705                 710                 715                 720
   Leu Val Glu Phe Asn Ser Ile Arg Asp Val Val Ser Leu Ile Pro Pro
   725 730 735
   Glu Glu Leu Lys Glu Trp Arg Ile Gly Thr Leu Asn Gly Phe Arg Met
   740 745 750
   Ser Pro Phe Val Glu Val Asp Glu Ser Phe Ala Lys Leu Leu Gly Tyr
   755 760 765
   Tyr Val Ser Glu Gly Tyr Ala Arg Lys Gln Arg Asn Pro Lys Asn Gly
   770 775 780
   Trp Ser Tyr Ser Val Lys Leu Tyr Asn Glu Asp Pro Glu Val Leu Asn
   785 790 795 800
   Asp Met Gly Lys Leu Ala Glu Arg Phe Phe Gly Lys Val Arg Lys Gly
   805 810 815
   Arg Asn Tyr Val Glu Ile Ser Arg Lys Met Gly Tyr Leu Leu Phe Glu
   820 825 830
   Ser Leu Cys Gly Val Leu Ala Lys Asn Lys Met Val Pro Glu Phe Ile
   835 840 845
   Phe Thr Phe Pro Thr Gly Val Arg Met Ala Phe Leu Glu Gly Tyr Phe
   850 855 860
   Ile Gly Asp Gly Asp Val His Pro Ser Lys Arg Leu Arg Leu Ser Thr
   865 870 875 880
   Lys Ser Glu Leu Leu Ala Asn Gln Leu Val Leu Leu Leu Asn Ser Val
   885 890 895
   Gly Val Ser Ala Val Lys Leu Gly His Asp Ser Ser Val Tyr Arg Val
   900 905 910
   Tyr Ile Asn Glu Ala Leu Pro Phe Val Lys Leu Asp Lys Lys Lys Asn
   915 920 925
   Ala Tyr Tyr Ser His Val Ile Pro Lys Glu Val Leu Ser Glu Ile Phe
   930 935 940
   Glu Lys Val Phe Gln Lys Asn Val Ser Pro Gln Thr Phe Arg Lys Met
   945 950 955 960
   Val Glu Gly Gly Lys Leu Asp Tyr Glu Lys Ala Gln Lys Leu Ser Trp
   965 970 975
   Leu Ile Asn Gly Asp Leu Val Leu Asp Arg Val Glu Ser Val Glu Ala
   980 985 990
   Glu Glu Tyr Ser Gly Tyr Val Tyr Asp Leu Ser Val Glu Asp Asn Glu
   995 1000 1005
   Asn Phe Leu Val Gly Phe Gly Leu Val Tyr Ala His Asn Ser Tyr
   1010 1015 1020
   Tyr Gly Tyr Tyr Gly Tyr Ala Arg Ala Arg Trp Tyr Cys Lys Glu
   1025 1030 1035
   Cys Ala Glu Ser Val Thr Ala Trp Gly Arg Glu Tyr Ile Glu Met
   1040 1045 1050
   Ser Ile Arg Glu Ile Glu Glu Lys Tyr Gly Phe Lys Val Leu Tyr
   1055 1060 1065
   Ala Asp Thr Asp Gly Phe His Ala Thr Ile Pro Gly Glu Asp Ala
   1070 1075 1080
   Glu Thr Ile Lys Lys Lys Ala Met Glu Phe Leu Lys Tyr Ile Asn
   1085 1090 1095
   Ser Lys Leu Pro Gly Ala Leu Glu Leu Glu Tyr Glu Gly Phe Tyr
   1100 1105 1110
   Arg Arg Gly Phe Phe Val Thr Lys Lys Lys Tyr Ala Val Ile Asp
   1115 1120 1125
   Glu Glu Gly Lys Ile Thr Thr Arg Gly Leu Glu Ile Val Arg Arg
   1130 1135 1140
   Asp Trp Ser Glu Ile Ala Lys Glu Thr Gln Ala Arg Val Leu Glu
   1145 1150 1155
   Ala Leu Leu Lys Asp Gly Asn Val Glu Glu Ala Val Ser Ile Val
   1160 1165 1170
   Lys Glu Val Thr Glu Lys Leu Ser Lys Tyr Glu Val Pro Pro Glu
   1175 1180 1185
   Lys Leu Val Ile His Glu Gln Ile Thr Arg Glu Leu Lys Asp Tyr
   1190 1195 1200
   Lys Ala Thr Gly Pro His Val Ala Ile Ala Lys Arg Leu Ala Ala
   1205 1210 1215
   Arg Gly Val Lys Ile Arg Pro Gly Thr Val Ile Ser Tyr Ile Val
   1220 1225 1230
   Leu Lys Gly Ser Gly Arg Ile Gly Asp Arg Ala Ile Pro Phe Asp
   1235 1240 1245
   Glu Phe Asp Pro Ala Lys His Arg Tyr Asp Ala Glu Tyr Tyr Ile
   1250 1255 1260
   Glu Asn Gln Val Leu Pro Ala Val Glu Arg Ile Leu Lys Ala Phe
       1265                1270                1275
   Gly Tyr Arg Lys Glu Asp Leu Arg Tyr Gln Lys Thr Arg Gln Val
       1280                1285                1290
   Gly Leu Gly Ala Trp Leu Lys Pro Lys Gly Lys Lys
       1295                1300                1305
<210> 3
   <211> 2328
   <212> DNA
   <213> Palaeococcus ferrophilus
<400> 3
   atgatcctcg atgctgacta cataaccgag aatggaaagc ccgtcgtgag gatattcaag      60
   aaggagaacg gcgagttcaa ggttgagtac gatagaaact ttgagcccta catctacgcc     120
   ctcctgaagg acgactccgc gattgaagaa atcaagaaga taaccgccga gaggcacgga     180
   acggtggtga gaattacaaa ggccgagaag gtggagagga agtttctcgg caggccggtt     240
   gaagtgtgga agctctactt cacccatcca caggacgtcc cggccataag ggataagata     300
   aggagccatc cggcagttgt ggacatctac gagtacgaca tacccttcgc gaagagatac     360
   ctcatcgaca agggcctggt tccgatggag ggggacgagg agctgaaaat gctcgccttc      420
   gacatcgaga cgctctatca cgagggcgag gagttcgccg agggacccat tctgatgata     480
   agctacgctg acgaaagtga ggctcgcgtc atcacctgga agaaggttga cctcccctac     540
   gtggatgccg tctcaaccga aaaagacatg ataaaggcct ttttgagggt cgtgaaggag      600
   aaggacccgg acgttctcat aacttacaac ggcgacaact tcgacttcgc ctatctaaaa     660
   aagcgctgcg aaaagctcgg ggtgaagttc atccttggaa gggatgggag cgagccgaag     720
   atccagagga tgggcgacag atttgcggtc gatgtgaagg gaaggataca cttcgatctc     780
   tatcccgtga taagaaggac gataaacctg ccgacctaca cgcttgaggc cgtctatgag     840
   gcgatatttg gaaggccgaa ggagaaggtc tacgcggagg agatagctca agcctgggaa     900
   accaacgagg gacttgagag ggtcgctcgc tactcaatgg aggatgccaa agtcacctac     960
   gagctgggaa aggagttctt cccgatggag gcccagcttt cccgtttgat cggccagccc    1020
   ctctgggacg tctcgcgctc cagcacgggc aatctggtcg agtggttcct ccttcggaag    1080
   gcctacgaga ggaacgaact ggccccaaac aagccctccg gaagggagta cgacgagagg    1140
   cgcggcggat acgctggcgg ctacgtgaag gagccggaga agggcctttg ggagaacata    1200
   gtgtatctag attacaaatc gttatatccc tcgataataa tcacccacaa cgtctcgccg    1260
   gatacgctca acagagaggg atgcaaggag tacgatgtgg ctcctcaggt cggccaccgc    1320
   ttctgcaagg acttcccggg cttcattcct agcctcctcg gagatcttct ggaggagagg    1380
   cagaagataa agaggaagat gaaggccact attgacccga tcgagaggag gctcctcgat    1440
   tacaggcagc gggcaatcaa gatcctggcg aacagttatt acggctacta cggctacgca    1500
   agggcccgct ggtactgcaa ggagtgcgcc gagagcgtca ccgcctgggg aagggagtac 1560
   atcgaaatga gcatacggga gatagaagag aaatacggct ttaaagtcct ctacgcggac 1620
   acggacggtt tccacgcgac gataccagga gaagatgccg agaccatcaa aaagaaggcc 1680
   atggagttcc tcaaatatat caactccaaa ctcccaggtg cgcttgagct cgagtacgag 1740
   ggcttctaca ggcgcggttt cttcgtcacc aagaagaagt acgcggtgat agacgaggag 1800
   ggcaagataa cgacgcgcgg gcttgagata gtcaggcgtg actggagcga gatagccaag 1860
   gagactcagg cgagggttct tgaggccctt ctcaaggacg gtaacgttga ggaggccgta 1920
   agcatagtca aagaagtgac ggagaagctg agcaagtacg aggttccgcc ggagaagctc 1980
   gttatccacg agcagataac gcgcgagctg aaggactaca aggcaacggg cccgcacgtg 2040
   gcgatagcga agaggttagc cgcgaggggc gtcaaaatcc gccccgggac ggtcatcagc 2100
   tacatagtcc tcaagggctc tggaaggata ggcgacaggg cgattccctt cgacgagttc 2160
   gacccggcca agcaccgcta cgacgctgaa tactacatcg agaaccaggt tctgccggcc 2220
   gttgagagga ttctaaaggc cttcggctat agaaaggagg atctgcgcta ccagaagacg 2280
   aggcaggttg ggcttggagc gtggttaaag ccgaagggga agaagtga 2328
<210> 4
   <211> 3918
   <212> DNA
   <213> Palaeococcus ferrophilus
<400> 4
   atgatcctcg atgctgacta cataaccgag aatggaaagc ccgtcgtgag gatattcaag 60
   aaggagaacg gcgagttcaa ggttgagtac gatagaaact ttgagcccta catctacgcc 120
   ctcctgaagg acgactccgc gattgaagaa atcaagaaga taaccgccga gaggcacgga 180
   acggtggtga gaattacaaa ggccgagaag gtggagagga agtttctcgg caggccggtt 240
   gaagtgtgga agctctactt cacccatcca caggacgtcc cggccataag ggataagata 300
   aggagccatc cggcagttgt ggacatctac gagtacgaca tacccttcgc gaagagatac 360
   ctcatcgaca agggcctggt tccgatggag ggggacgagg agctgaaaat gctcgccttc 420
   gacatcgaga cgctctatca cgagggcgag gagttcgccg agggacccat tctgatgata 480
   agctacgctg acgaaagtga ggctcgcgtc atcacctgga agaaggttga cctcccctac 540
   gtggatgccg tctcaaccga aaaagacatg ataaaggcct ttttgagggt cgtgaaggag 600
   aaggacccgg acgttctcat aacttacaac ggcgacaact tcgacttcgc ctatctaaaa 660
   aagcgctgcg aaaagctcgg ggtgaagttc atccttggaa gggatgggag cgagccgaag 720
   atccagagga tgggcgacag atttgcggtc gatgtgaagg gaaggataca cttcgatctc 780
   tatcccgtga taagaaggac gataaacctg ccgacctaca cgcttgaggc cgtctatgag 840
   gcgatatttg gaaggccgaa ggagaaggtc tacgcggagg agatagctca agcctgggaa 900
   accaacgagg gacttgagag ggtcgctcgc tactcaatgg aggatgccaa agtcacctac 960
   gagctgggaa aggagttctt cccgatggag gcccagcttt cccgtttgat cggccagccc 1020
   ctctgggacg tctcgcgctc cagcacgggc aatctggtcg agtggttcct ccttcggaag 1080
   gcctacgaga ggaacgaact ggccccaaac aagccctccg gaagggagta cgacgagagg 1140
   cgcggcggat acgctggcgg ctacgtgaag gagccggaga agggcctttg ggagaacata 1200
   gtgtatctag attacaaatc gttatatccc tcgataataa tcacccacaa cgtctcgccg 1260
   gatacgctca acagagaggg atgcaaggag tacgatgtgg ctcctcaggt cggccaccgc 1320
   ttctgcaagg acttcccggg cttcattcct agcctcctcg gagatcttct ggaggagagg 1380
   cagaagataa agaggaagat gaaggccact attgacccga tcgagaggag gctcctcgat 1440
   tacaggcagc gggcaatcaa gatcctggcg aacagtattc ttcccgacga gtggcttccc 1500
   attattgaaa acgggacggt tcgcttcgtc aggattgggg agttcataga ctggaaaatg 1560
   gatgaaaacg ctgaaagagt gcatagggaa ggggaaacgg aaatccttga agtcagtggt 1620
   cttgaagtcc aatccttcaa cagggaaacg aagaaggccg agcttaagag ggtaaaggcc 1680
   ctaatcaggc accgctattc gggcaaagct tacaacataa aactgaagtc tgggaggaga 1740
   ataaagataa cctctggcca cagcctcttc gttgaggtca cgggggatga actcaagccc 1800
   ggcgacctgg ttgcagtccc gcggagggtg aagcttccgg agagaaacca cgtgttgaac 1860
   ctcgtggagc ttctcctcgg attccctgag gacgaaacgt cagacattgt tatgacaatt 1920
   cccgtcaaag agcggaagaa cttctttaaa ggaatgctta gaaccctgcg ctggattttt 1980
   ggggaggaga aaaggccaag gacagcaagg cgttatctca agcaccttga ggatctgggt 2040
   tatgtcaggc ttaagaagat aggctatgaa gtacttgact gggaggcact taggaaatac 2100
   agaaggctct acgaggcact tgtcgaaaaa atcagataca acggcaacaa gagagagtac 2160
   ctcgttgagt tcaactccat ccgggacgtg gtaagcctaa ttcccccgga agagcttaag 2220
   gagtggagaa ttggaacgct gaacggcttt agaatgagtc cttttgtgga ggttgacgag 2280
   tccttcgcaa agctcctcgg ctactatgtg agcgagggct atgcaagaaa gcagagaaat 2340
   cccaaaaacg gctggagcta cagcgtgaag ctctacaacg aagaccctga agtactgaac 2400
   gatatgggga agctcgctga gaggttcttt ggaaaggtta gaaaaggccg gaactacgtt 2460
   gaaatatcga ggaagatggg ctacctgctc tttgagagcc tttgcggtgt tctggcgaaa 2520
   aacaagatgg ttccagagtt catcttcacg tttccgacag gggttaggat ggctttcctt 2580
   gaggggtact tcatcggcga tggcgacgtc cacccgagca aaaggctcag gctctccacg 2640
   aagagcgagc ttttggccaa ccagctcgtc ctcctcttga actctgtggg agtttcggcc 2700
   gtaaagctcg ggcacgacag cagcgtttac agggtttaca taaacgaggc gctcccgttc 2760
   gtaaagctgg acaagaaaaa gaacgcctac tattcgcacg tgatccccaa ggaagtcctg 2820
   agcgagatct ttgagaaggt cttccagaag aacgtcagtc ctcagacctt caggaagatg 2880
   gttgaaggcg gaaagctcga ttatgaaaag gcccaaaaac tctcctggct cattaatggc 2940
   gatctagtgc ttgaccgtgt tgagtccgtt gaggctgagg aatacagcgg ctatgtctac 3000
   gacctgagcg tcgaagacaa cgagaacttc ctcgttggtt ttgggttggt ctatgctcac 3060
   aacagttatt acggctacta cggctacgca agggcccgct ggtactgcaa ggagtgcgcc 3120
   gagagcgtca ccgcctgggg aagggagtac atcgaaatga gcatacggga gatagaagag     3180
   aaatacggct ttaaagtcct ctacgcggac acggacggtt tccacgcgac gataccagga     3240
   gaagatgccg agaccatcaa aaagaaggcc atggagttcc tcaaatatat caactccaaa     3300
   ctcccaggtg cgcttgagct cgagtacgag ggcttctaca ggcgcggttt cttcgtcacc     3360
   aagaagaagt acgcggtgat agacgaggag ggcaagataa cgacgcgcgg gcttgagata     3420
   gtcaggcgtg actggagcga gatagccaag gagactcagg cgagggttct tgaggccctt     3480
   ctcaaggacg gtaacgttga ggaggccgta agcatagtca aagaagtgac ggagaagctg     3540
   agcaagtacg aggttccgcc ggagaagctc gttatccacg agcagataac gcgcgagctg     3600
   aaggactaca aggcaacggg cccgcacgtg gcgatagcga agaggttagc cgcgaggggc     3660
   gtcaaaatcc gccccgggac ggtcatcagc tacatagtcc tcaagggctc tggaaggata     3720
   ggcgacaggg cgattccctt cgacgagttc gacccggcca agcaccgcta cgacgctgaa     3780
   tactacatcg agaaccaggt tctgccggcc gttgagagga ttctaaaggc cttcggctat     3840
   agaaaggagg atctgcgcta ccagaagacg aggcaggttg ggcttggagc gtggttaaag     3900
   ccgaagggga agaagtga                                                   3918
<210> 5
   <211> 29
   <212> DNA
   <213> Artificial
<220>
   <223> Primer sequence
<220>
   <221> misc_feature
   <222> (18)..(18)
   <223> n is a, c, g, or t
<400> 5
   cgcgggagaa cctggttntc datrtarta 29
<210> 6
   <211> 9
   <212> PRT
   <213> Unknown
<220>
   <223> Protein fragment
<400> 6
   Tyr Tyr Ile Glu Asn Gln Val Leu Pro
   1               5
<210> 7
   <211> 29
   <212> DNA
   <213> Artificial
<220>
   <223> Primer sequence
<220>
   <221> misc_feature
   <222> (21)..(21)
   <223> n is a, c, g, or t
<400> 7
   tactacggat aggccaargc nagrtggta 29
<210> 8
   <211> 9
   <212> PRT
   <213> Unknown
<220>
   <223> Protein fragment
<220>
   <221> MISC_FEATURE
   <222> (4)..(4)
   <223> Xaa is STOP
<400> 8
   Tyr Tyr Gly Xaa Ala Lys Ala Arg Trp
   1 5
<210> 9
   <211> 18
   <212> DNA
   <213> Artificial
<220>
   <223> Primer sequence
<400> 9
   tgtaaaacga cggccagt 18
<210> 10
   <211> 24
   <212> DNA
   <213> Artificial
<220>
   <223> Primer sequence
<400> 10
   agcggataac aatttcacac agga 24
<210> 11
   <211> 20
   <212> DNA
   <213> Artificial
<220>
   <223> Primer sequence
<400> 11
   tatctcgctc cagtcacgcc 20
<210> 12
   <211> 23
   <212> DNA
   <213> Artificial
<220>
   <223> Primer sequence
<220>
   <221> misc_feature
   <222> (15)..(15)
   <223> n is a, c, g, or t
<400> 12
   agggagttct tcccnatgga rgc 23
<210> 13
   <211> 8
   <212> PRT
   <213> Unknown
<220>
   <223> Protein fragment
<400> 13
   Lys Glu Phe Phe Pro Met Glu Ala
   1               5
<210> 14
   <211> 19
   <212> DNA
   <213> Artificial
<220>
   <223> Primer sequence
<400> 14
   aacaccgcaa aggctctca 19
<210> 15
   <211> 19
   <212> DNA
   <213> Artificial
<220>
   <223> Primer sequence
<400> 15
   gcttgagttc atcccccgt 19
<210> 16
   <211> 20
   <212> DNA
   <213> Artificial
<220>
   <223> Primer sequence
<400> 16
   tcaggcgagg gttcttgagg 20
<210> 17
   <211> 20
   <212> DNA
   <213> Artificial
<220>
   <223> Primer sequence
<400> 17
   cgtggcgata gcgaagaggt 20
<210> 18
   <211> 20
   <212> DNA
   <213> Artificial
<220>
   <223> Primer sequence
<400> 18
   tatctcgctc cagtcacgcc 20
<210> 19
   <211> 20
   <212> DNA
   <213> Artificial
<220>
   <223> Primer sequence
<400> 19
   cgctggcggc tacgtgaagg 20
<210> 20
   <211> 20
   <212> DNA
   <213> Artificial
<220>
   <223> Primer sequence
<400> 20
   tctcgtcgta ctcccttccg 20
<210> 21
   <211> 18
   <212> DNA
   <213> Artificial
<220>
   <223> Primer sequence
<400> 21
   gtttggggcc agttcgtt 18
<210> 22
   <211> 20
   <212> DNA
   <213> Artificial
<220>
   <223> Primer sequence
<400> 22
   aaagtgaggc tcgcgtcatc 20
<210> 23
   <211> 20
   <212> DNA
   <213> Artificial
<220>
   <223> Primer sequence
<400> 23
   actcctcgcc ctcgtgatag 20
<210> 24
   <211> 20
   <212> DNA
   <213> Artificial
<220>
   <223> Primer sequence
<400> 24
   attttcagct cctcgtcccc 20
<210> 25
   <211> 44
   <212> DNA
   <213> Artificial
<220>
   <223> Primer sequence
<400> 25
   aagcttcata tgatcctgga tgctgactac ataaccgaga atgg 44
<210> 26
   <211> 39
   <212> DNA
   <213> Artificial
<220>
   <223> Primer sequence
<400> 26
   gaattcgtcg acttacttct tccccttcgg ctttaacca 39
<210> 27
   <211> 55
   <212> DNA
   <213> Artificial
<220>
   <223> Primer sequence
<400> 27
   ttcccctcta gaaataattt tgtttaactt taagaaggag atatactatg cacca 55
<210> 28
   <211> 55
   <212> DNA
   <213> Artificial
<220>
   <223> Primer sequence
<400> 28
   gaattcggat ccgctagcca tatggtgatg gtgatggtgc atagtatatc tcctt 55
<210> 29
   <211> 24
   <212> DNA
   <213> Artificial
<220>
   <223> Primer sequence
<400> 29
   aaattaatac gactcactat aggg 24
<210> 30
   <211> 19
   <212> DNA
   <213> Artificial
<220>
   <223> Primer sequence
<400> 30
   gctagttatt gctcagcgg 19
<210> 31
   <211> 36
   <212> DNA
   <213> Artificial
<220>
   <223> Primer sequence
<400> 31
   aagtttgaat tcgccaggat cttgattgcc cgctgc 36
<210> 32
   <211> 39
   <212> DNA
   <213> Artificial
<220>
   <223> Primer sequence
<400> 32
   cctggcgaat tcttattacg gctactacgg ctacgcaag 39
<210> 33
   <211> 20
   <212> DNA
   <213> Artificial
<220>
   <223> Primer sequence
<400> 33
   cctgctctgc cgcttcacgc 20
<210> 34
   <211> 24
   <212> DNA
   <213> Artificial
<220>
   <223> Primer sequence
<400> 34
   ccatgattca gtgtgcccgt ctgg 24
<210> 35
   <211> 20
   <212> PRT
   <213> Palaeococcus ferrophilus
<400> 35
   Arg Gln Arg Ala Ile Lys Ile Leu Ala Asn Ser Tyr Tyr Gly Tyr Tyr
   1               5                   10                  15
   Gly Tyr Ala Arg
               20
<210> 36
   <211> 23
   <212> PRT
   <213> Artificial
<220>
   <223> Consensus sequence
<220>
   <221> MISC_FEATURE
   <222> (2)..(2)
   <223> Xaa is Lys or Arg
<220>
   <221> MISC_FEATURE
   <222> (3)..(3)
   <223> Xaa is Glu or Asp
<220>
   <221> MISC_FEATURE
   <222> (4)..(4)
   <223> Xaa is Met or Ala
<220>
   <221> MISC_FEATURE
   <222> (7)..(7)
   <223> Xaa is Arg or Ala
<220>
   <221> MISC_FEATURE
   <222> (10)..(10)
   <223> Xaa is Lys, Arg or His
<220>
   <221> MISC_FEATURE
   <222> (11)..(11)
   <223> Xaa is Val or Ile
<220>
   <221> MISC_FEATURE
   <222> (12)..(12)
   <223> Xaa is val, Ile or Lys
<220>
   <221> MISC_FEATURE
   <222> (13)..(13)
   <223> Xaa is Lys or Arg
<220>
   <221> MISC_FEATURE
   <222> (19)..(19)
   <223> Xaa is val or Ile
<220>
   <221> MISC_FEATURE
   <222> (20)..(20)
   <223> Xaa is Leu or Ile
<220>
   <221> MISC_FEATURE
   <222> (21)..(21)
   <223> Xaa is val or Ile
<400> 36
   Glu Xaa Xaa Xaa Ile Lys Xaa Phe Leu Xaa Xaa Xaa Xaa Glu Lys Asp
   1               5                   10                  15
   Pro Asp Xaa Xaa Xaa Thr Tyr
               20
<210> 37
   <211> 36
   <212> PRT
   <213> Artificial
<220>
   <223> Consensus sequence
<220>
   <221> MISC_FEATURE
   <222> (2)..(2)
   <223> Xaa is Tyr or Phe
<220>
   <221> MISC_FEATURE
   <222> (8)..(8)
   <223> Xaa is Lys or Arg
<220>
   <221> MISC_FEATURE
   <222> (12)..(12)
   <223> Xaa is Glu or Asp
<220>
   <221> MISC_FEATURE
   <222> (13)..(13)
   <223> Xaa is Arg, Lys or Thr
<220>
   <221> MISC_FEATURE
   <222> (14)..(14)
   <223> Xaa is Leu or Ile
<220>
   <221> MISC_FEATURE
   <222> (15)..(15)
   <223> Xaa is Phe or Leu
<220>
   <221> MISC_FEATURE
   <222> (16)..(16)
   <223> Xaa is Tyr or Ser
<220>
   <221> MISC_FEATURE
   <222> (19)..(19)
   <223> Xaa is Tyr or Phe
<220>
   <221> MISC_FEATURE
   <222> (20)..(20)
   <223> Xaa is Lys or Arg
<220>
   <221> MISC_FEATURE
   <222> (21)..(21)
   <223> Xaa is Ser or Ala
<220>
   <221> MISC_FEATURE
   <222> (28)..(28)
   <223> Xaa is Val or Ile
<400> 37
   Gly Xaa Val Lys Glu Pro Glu Xaa Gly Leu Trp Xaa Xaa Xaa Xaa Xaa
   1               5                   10                  15
   Leu Asp Xaa Xaa Xaa Leu Tyr Pro Ser Ile Ile Xaa Thr His Asn Val
               20                  25                  30
   Ser Pro Asp Thr
           35
<210> 38
   <211> 22
   <212> PRT
   <213> Artificial
<220>
   <223> Consensus sequence
<220>
   <221> MISC_FEATURE
   <222> (6)..(6)
   <223> Xaa is Leu or Ile
<220>
   <221> MISC_FEATURE
   <222> (8)..(8)
   <223> Xaa is Gly, Lys or Glu
<220>
   <221> MISC_FEATURE
   <222> (9)..(9)
   <223> Xaa is Asn, Asp, His or Glu
<220>
   <221> MISC_FEATURE
   <222> (11)..(11)
   <223> Xaa is Leu or Ile
<220>
   <221> MISC_FEATURE
   <222> (12)..(12)
   <223> Xaa is Glu or Asp
<220>
   <221> MISC_FEATURE
   <222> (13)..(13)
   <223> Xaa is Glu or Thr
<220>
   <221> MISC_FEATURE
   <222> (16)..(16)
   <223> Xaa is Tyr, Phe or His
<220>
   <221> MISC_FEATURE
   <222> (17)..(17)
   <223> Xaa is Val or Ile
<220>
   <221> MISC_FEATURE
   <222> (19)..(19)
   <223> Xaa is Arg, Lys or Thr
<400> 38
   Gly Phe Ile Pro Ser Xaa Leu Xaa Xaa Leu Xaa Xaa Xaa Arg Gln Xaa
   1               5                   10                  15
   Xaa Lys Xaa Lys Met Lys
               20
<210> 39
   <211> 22
   <212> PRT
   <213> Artificial
<220>
   <223> Consensus sequence
<220>
   <221> MISC_FEATURE
   <222> (5)..(5)
   <223> Xaa is Lys or Arg
<220>
   <221> MISC_FEATURE
   <222> (7)..(7)
   <223> Xaa is Leu or Ile
<220>
   <221> MISC_FEATURE
   <222> (9)..(9)
   <223> Xaa is Leu or Ile
<220>
   <221> MISC_FEATURE
   <222> (14)..(14)
   <223> Xaa is Tyr or Phe
<220>
   <221> MISC_FEATURE
   <222> (18)..(18)
   <223> Xaa is Tyr or Thr
<220>
   <221> MISC_FEATURE
   <222> (21)..(21)
   <223> Xaa is Ala or Pro
<220>
   <221> MISC_FEATURE
   <222> (22)..(22)
   <223> Xaa is Lys or Arg
<400> 39
   Asp Tyr Arg Gln Xaa Ala Xaa Lys Xaa Leu Ala Asn Ser Xaa Tyr Gly
   1               5                   10                  15
   Tyr Xaa Gly Tyr Xaa Xaa
               20
<210> 40
   <211> 7
   <212> PRT
   <213> Artificial
<220>
   <223> Consensus sequence
<220>
   <221> MISC_FEATURE
   <222> (5)..(5)
   <223> Xaa is Phe or Leu
<220>
   <221> MISC_FEATURE
   <222> (6)..(6)
   <223> Xaa is Tyr, Phe or His
<400> 40
   Asp Thr Asp Gly Xaa Xaa Ala
   1               5
<210> 41
   <211> 23
   <212> PRT
   <213> Artificial
<220>
   <223> Consensus sequence
<220>
   <221> MISC_FEATURE
   <222> (5)..(5)
   <223> Xaa is Gly or His
<220>
   <221> MISC_FEATURE
   <222> (6)..(6)
   <223> Xaa is val or Ile
<220>
   <221> MISC_FEATURE
   <222> (7)..(7)
   <223> Xaa is Val, Thr or Ile
<220>
   <221> MISC_FEATURE
   <222> (13)..(13)
   <223> Xaa is val or Ile
<220>
   <221> MISC_FEATURE
   <222> (20)..(20)
   <223> Xaa is Glu or Asp
<400> 41
   Asp Glu Glu Gly Xaa Xaa Xaa Thr Arg Gly Leu Glu Xaa Val Arg Arg
   1               5                   10                  15
   Asp Trp Ser Xaa Ile Ala Lys
               20
<210> 42
   <211> 69
   <212> DNA
   <213> Artificial
<220>
   <223> Plasmid fragment
<400> 42
   tctagaaata attttgttta actttaagaa ggagatatac tatgcaccat caccatcacc      60
   atatggcta                                                              69
<210> 43
   <211> 8
   <212> PRT
   <213> Artificial
<220>
   <223> Protein tag sequence
<400> 43
   Met His His His His His His Met
   1               5
<210> 44
   <211> 15
   <212> PRT
   <213> Artificial
<220>
   <223> Consensus sequence
<220>
   <221> MISC_FEATURE
   <222> (7)..(7)
   <223> Xaa is val or Ile
<400> 44
   Leu Tyr Pro Ser Ile Ile Xaa Thr His Asn Val Ser Pro Asp Thr
   1               5                   10                  15
<210> 45
   <211> 16
   <212> PRT
   <213> Artificial
<220>
   <223> Consensus sequence
<220>
   <221> MISC_FEATURE
   <222> (6)..(6)
   <223> Xaa is Val or Ile
<220>
   <221> MISC_FEATURE
   <222> (13)..(13)
   <223> Xaa is Glu or Asp
<400> 45
   Thr Arg Gly Leu Glu Xaa Val Arg Arg Asp Trp Ser Xaa Ile Ala Lys
   1               5                   10                  15
<210> 46
   <211> 842
   <212> PRT
   <213> Artificial
<220>
   <223> Chimeric protein
<400> 46
   Met Ile Leu Asp Ala Asp Tyr Ile Thr Glu Asn Gly Lys Pro Val Val
   1               5                   10                  15
   Arg Ile Phe Lys Lys Glu Asn Gly Glu Phe Lys Val Glu Tyr Asp Arg
               20                  25                  30
   Asn Phe Glu Pro Tyr Ile Tyr Ala Leu Leu Lys Asp Asp Ser Ala Ile
           35                  40                  45
   Glu Glu Ile Lys Lys Ile Thr Ala Glu Arg His Gly Thr Val Val Arg
       50                  55                  60
   Ile Thr Lys Ala Glu Lys Val Glu Arg Lys Phe Leu Gly Arg Pro Val
   65                  70                  75                  80
   Glu Val Trp Lys Leu Tyr Phe Thr His Pro Gln Asp Val Pro Ala Ile
                   85                  90                  95
   Arg Asp Lys Ile Arg Ser His Pro Ala Val Val Asp Ile Tyr Glu Tyr
               100                 105                 110
   Asp Ile Pro Phe Ala Lys Arg Tyr Leu Ile Asp Lys Gly Leu Val Pro
           115                 120                 125
   Met Glu Gly Asp Glu Glu Leu Lys Met Leu Ala Phe Asp Ile Glu Thr
       130                 135                 140
   Leu Tyr His Glu Gly Glu Glu Phe Ala Glu Gly Pro Ile Leu Met Ile
   145                 150                 155                 160
   Ser Tyr Ala Asp Glu Ser Glu Ala Arg Val Ile Thr Trp Lys Lys Val
   165 170 175
   Asp Leu Pro Tyr Val Asp Ala Val Ser Thr Glu Lys Asp Met Ile Lys
   180 185 190
   Ala Phe Leu Arg Val Val Lys Glu Lys Asp Pro Asp Val Leu Ile Thr
   195 200 205
   Tyr Asn Gly Asp Asn Phe Asp Phe Ala Tyr Leu Lys Lys Arg Cys Glu
   210 215 220
   Lys Leu Gly Val Lys Phe Ile Leu Gly Arg Asp Gly Ser Glu Pro Lys
   225 230 235 240
   Ile Gln Arg Met Gly Asp Arg Phe Ala Val Asp Val Lys Gly Arg Ile
   245 250 255
   His Phe Asp Leu Tyr Pro Val Ile Arg Arg Thr Ile Asn Leu Pro Thr
   260 265 270
   Tyr Thr Leu Glu Ala Val Tyr Glu Ala Ile Phe Gly Arg Pro Lys Glu
   275 280 285
   Lys Val Tyr Ala Glu Glu Ile Ala Gln Ala Trp Glu Thr Asn Glu Gly
   290 295 300
   Leu Glu Arg Val Ala Arg Tyr Ser Met Glu Asp Ala Lys Val Thr Tyr
   305 310 315 320
   Glu Leu Gly Lys Glu Phe Phe Pro Met Glu Ala Gln Leu Ser Arg Leu
   325 330 335
   Ile Gly Gln Pro Leu Trp Asp Val Ser Arg Ser Ser Thr Gly Asn Leu
   340 345 350
   Val Glu Trp Phe Leu Leu Arg Lys Ala Tyr Glu Arg Asn Glu Leu Ala
   355 360 365
   Pro Asn Lys Pro Ser Gly Arg Glu Tyr Asp Glu Arg Arg Gly Gly Tyr
   370 375 380
   Ala Gly Gly Tyr Val Lys Glu Pro Glu Lys Gly Leu Trp Glu Asn Ile
   385 390 395 400
   Val Tyr Leu Asp Tyr Lys Ser Leu Tyr Pro Ser Ile Ile Ile Thr His
   405 410 415
   Asn Val Ser Pro Asp Thr Leu Asn Arg Glu Gly Cys Lys Glu Tyr Asp
   420 425 430
   Val Ala Pro Gln Val Gly His Arg Phe Cys Lys Asp Phe Pro Gly Phe
   435 440 445
   Ile Pro Ser Leu Leu Gly Asp Leu Leu Glu Glu Arg Gln Lys Ile Lys
   450 455 460
   Arg Lys Met Lys Ala Thr Ile Asp Pro Ile Glu Arg Arg Leu Leu Asp
   465 470 475 480
   Tyr Arg Gln Arg Ala Ile Lys Ile Leu Ala Asn Ser Tyr Tyr Gly Tyr
   485 490 495
   Tyr Gly Tyr Ala Arg Ala Arg Trp Tyr Cys Lys Glu Cys Ala Glu Ser
   500 505 510
   Val Thr Ala Trp Gly Arg Glu Tyr Ile Glu Met Ser Ile Arg Glu Ile
   515 520 525
   Glu Glu Lys Tyr Gly Phe Lys Val Leu Tyr Ala Asp Thr Asp Gly Phe
   530 535 540
   His Ala Thr Ile Pro Gly Glu Asp Ala Glu Thr Ile Lys Lys Lys Ala
   545 550 555 560
   Met Glu Phe Leu Lys Tyr Ile Asn Ser Lys Leu Pro Gly Ala Leu Glu
   565 570 575
   Leu Glu Tyr Glu Gly Phe Tyr Arg Arg Gly Phe Phe Val Thr Lys Lys
   580 585 590
   Lys Tyr Ala Val Ile Asp Glu Glu Gly Lys Ile Thr Thr Arg Gly Leu
   595 600 605
   Glu Ile Val Arg Arg Asp Trp Ser Glu Ile Ala Lys Glu Thr Gln Ala
   610 615 620
   Arg Val Leu Glu Ala Leu Leu Lys Asp Gly Asn Val Glu Glu Ala Val
   625 630 635 640
   Ser Ile Val Lys Glu Val Thr Glu Lys Leu Ser Lys Tyr Glu Val Pro
   645 650 655
   Pro Glu Lys Leu Val Ile His Glu Gln Ile Thr Arg Glu Leu Lys Asp
   660 665 670
   Tyr Lys Ala Thr Gly Pro His Val Ala Ile Ala Lys Arg Leu Ala Ala
   675 680 685
   Arg Gly Val Lys Ile Arg Pro Gly Thr Val Ile Ser Tyr Ile Val Leu
   690 695 700
   Lys Gly Ser Gly Arg Ile Gly Asp Arg Ala Ile Pro Phe Asp Glu Phe
   705 710 715 720
   Asp Pro Ala Lys His Arg Tyr Asp Ala Glu Tyr Tyr Ile Glu Asn Gln
                   725                 730                 735
   Val Leu Pro Ala Val Glu Arg Ile Leu Lys Ala Phe Gly Tyr Arg Lys
               740                 745                 750
   Glu Asp Leu Arg Tyr Gln Lys Thr Arg Gln Val Gly Leu Gly Ala Trp
           755                 760                 765
   Leu Lys Pro Lys Gly Lys Lys Gly Ser Gly Thr His Met Ala Cys Glu
       770                 775                 780
   Lys Pro Val Lys Val Arg Asp Pro Thr Thr Gly Lys Glu Val Glu Leu
   785                 790                 795                 800
   Val Pro Ile Lys Val Trp Gln Leu Ala Pro Arg Gly Arg Lys Gly Val
                   805                 810                 815
   Lys Ile Gly Leu Phe Lys Ser Pro Glu Thr Gly Lys Tyr Phe Arg Ala
               820                 825                 830
   Lys Val Pro Asp Asp Tyr Pro Ile Cys Ser
           835                 840
<210> 47
   <211> 2529
   <212> DNA
   <213> Artificial
<220>
   <223> cloned construct
<400> 47
   atgatcctcg atgctgacta cataaccgag aatggaaagc ccgtcgtgag gatattcaag      60
   aaggagaacg gcgagttcaa ggttgagtac gatagaaact ttgagcccta catctacgcc     120
   ctcctgaagg acgactccgc gattgaagaa atcaagaaga taaccgccga gaggcacgga     180
   acggtggtga gaattacaaa ggccgagaag gtggagagga agtttctcgg caggccggtt     240
   gaagtgtgga agctctactt cacccatcca caggacgtcc cggccataag ggataagata     300
   aggagccatc cggcagttgt ggacatctac gagtacgaca tacccttcgc gaagagatac     360
   ctcatcgaca agggcctggt tccgatggag ggggacgagg agctgaaaat gctcgccttc     420
   gacatcgaga cgctctatca cgagggcgag gagttcgccg agggacccat tctgatgata     480
   agctacgctg acgaaagtga ggctcgcgtc atcacctgga agaaggttga cctcccctac     540
   gtggatgccg tctcaaccga aaaagacatg ataaaggcct ttttgagggt cgtgaaggag      600
   aaggacccgg acgttctcat aacttacaac ggcgacaact tcgacttcgc ctatctaaaa     660
   aagcgctgcg aaaagctcgg ggtgaagttc atccttggaa gggatgggag cgagccgaag     720
   atccagagga tgggcgacag atttgcggtc gatgtgaagg gaaggataca cttcgatctc     780
   tatcccgtga taagaaggac gataaacctg ccgacctaca cgcttgaggc cgtctatgag 840
   gcgatatttg gaaggccgaa ggagaaggtc tacgcggagg agatagctca agcctgggaa 900
   accaacgagg gacttgagag ggtcgctcgc tactcaatgg aggatgccaa agtcacctac 960
   gagctgggaa aggagttctt cccgatggag gcccagcttt cccgtttgat cggccagccc 1020
   ctctgggacg tctcgcgctc cagcacgggc aatctggtcg agtggttcct ccttcggaag 1080
   gcctacgaga ggaacgaact ggccccaaac aagccctccg gaagggagta cgacgagagg 1140
   cgcggcggat acgctggcgg ctacgtgaag gagccggaga agggcctttg ggagaacata 1200
   gtgtatctag attacaaatc gttatatccc tcgataataa tcacccacaa cgtctcgccg 1260
   gatacgctca acagagaggg atgcaaggag tacgatgtgg ctcctcaggt cggccaccgc 1320
   ttctgcaagg acttcccggg cttcattcct agcctcctcg gagatcttct ggaggagagg 1380
   cagaagataa agaggaagat gaaggccact attgacccga tcgagaggag gctcctcgat 1440
   tacaggcagc gggcaatcaa gatcctggcg aacagttatt acggctacta cggctacgca 1500
   agggcccgct ggtactgcaa ggagtgcgcc gagagcgtca ccgcctgggg aagggagtac 1560
   atcgaaatga gcatacggga gatagaagag aaatacggct ttaaagtcct ctacgcggac 1620
   acggacggtt tccacgcgac gataccagga gaagatgccg agaccatcaa aaagaaggcc 1680
   atggagttcc tcaaatatat caactccaaa ctcccaggtg cgcttgagct cgagtacgag 1740
   ggcttctaca ggcgcggttt cttcgtcacc aagaagaagt acgcggtgat agacgaggag 1800
   ggcaagataa cgacgcgcgg gcttgagata gtcaggcgtg actggagcga gatagccaag 1860
   gagactcagg cgagggttct tgaggccctt ctcaaggacg gtaacgttga ggaggccgta 1920
   agcatagtca aagaagtgac ggagaagctg agcaagtacg aggttccgcc ggagaagctc 1980
   gttatccacg agcagataac gcgcgagctg aaggactaca aggcaacggg cccgcacgtg 2040
   gcgatagcga agaggttagc cgcgaggggc gtcaaaatcc gccccgggac ggtcatcagc 2100
   tacatagtcc tcaagggctc tggaaggata ggcgacaggg cgattccctt cgacgagttc 2160
   gacccggcca agcaccgcta cgacgctgaa tactacatcg agaaccaggt tctgccggcc 2220
   gttgagagga ttctaaaggc cttcggctat agaaaggagg atctgcgcta ccagaagacg 2280
   aggcaggttg ggcttggagc gtggttaaag ccgaagggga agaagggatc cggaacacac 2340
   atggcgtgtg agaagcctgt taaggttcgt gaccctacta ctggtaagga ggtagagctg 2400
   gtaccaatca aggtgtggca gctagcaccc aggggtagga agggcgtcaa gataggccta 2460
   ttcaagagcc ccgaaacagg caagtacttc agagccaagg taccagacga ctacccaatc 2520
   tgcagctaa 2529
<210> 48
   <211> 45
   <212> DNA
   <213> Artificial
<220>
   <223> Primer sequence
<400> 48
   gaattcgtcg acttaggatc ccttcttccc cttcggcttt aacca 45
<210> 49
   <211> 2334
   <212> DNA
   <213> Artificial
<220>
   <223> cloned construct
<400> 49
   atgatcctcg atgctgacta cataaccgag aatggaaagc ccgtcgtgag gatattcaag      60
   aaggagaacg gcgagttcaa ggttgagtac gatagaaact ttgagcccta catctacgcc     120
   ctcctgaagg acgactccgc gattgaagaa atcaagaaga taaccgccga gaggcacgga     180
   acggtggtga gaattacaaa ggccgagaag gtggagagga agtttctcgg caggccggtt     240
   gaagtgtgga agctctactt cacccatcca caggacgtcc cggccataag ggataagata     300
   aggagccatc cggcagttgt ggacatctac gagtacgaca tacccttcgc gaagagatac     360
   ctcatcgaca agggcctggt tccgatggag ggggacgagg agctgaaaat gctcgccttc     420
   gacatcgaga cgctctatca cgagggcgag gagttcgccg agggacccat tctgatgata     480
   agctacgctg acgaaagtga ggctcgcgtc atcacctgga agaaggttga cctcccctac     540
   gtggatgccg tctcaaccga aaaagacatg ataaaggcct ttttgagggt cgtgaaggag     600
   aaggacccgg acgttctcat aacttacaac ggcgacaact tcgacttcgc ctatctaaaa     660
   aagcgctgcg aaaagctcgg ggtgaagttc atccttggaa gggatgggag cgagccgaag     720
   atccagagga tgggcgacag atttgcggtc gatgtgaagg gaaggataca cttcgatctc     780
   tatcccgtga taagaaggac gataaacctg ccgacctaca cgcttgaggc cgtctatgag     840
   gcgatatttg gaaggccgaa ggagaaggtc tacgcggagg agatagctca agcctgggaa     900
   accaacgagg gacttgagag ggtcgctcgc tactcaatgg aggatgccaa agtcacctac     960
   gagctgggaa aggagttctt cccgatggag gcccagcttt cccgtttgat cggccagccc    1020
   ctctgggacg tctcgcgctc cagcacgggc aatctggtcg agtggttcct ccttcggaag    1080
   gcctacgaga ggaacgaact ggccccaaac aagccctccg gaagggagta cgacgagagg    1140
   cgcggcggat acgctggcgg ctacgtgaag gagccggaga agggcctttg ggagaacata    1200
   gtgtatctag attacaaatc gttatatccc tcgataataa tcacccacaa cgtctcgccg    1260
   gatacgctca acagagaggg atgcaaggag tacgatgtgg ctcctcaggt cggccaccgc    1320
   ttctgcaagg acttcccggg cttcattcct agcctcctcg gagatcttct ggaggagagg    1380
   cagaagataa agaggaagat gaaggccact attgacccga tcgagaggag gctcctcgat    1440
   tacaggcagc gggcaatcaa gatcctggcg aacagttatt acggctacta cggctacgca    1500
   agggcccgct ggtactgcaa ggagtgcgcc gagagcgtca ccgcctgggg aagggagtac    1560
   atcgaaatga gcatacggga gatagaagag aaatacggct ttaaagtcct ctacgcggac    1620
   acggacggtt tccacgcgac gataccagga gaagatgccg agaccatcaa aaagaaggcc    1680
   atggagttcc tcaaatatat caactccaaa ctcccaggtg cgcttgagct cgagtacgag     1740
   ggcttctaca ggcgcggttt cttcgtcacc aagaagaagt acgcggtgat agacgaggag     1800
   ggcaagataa cgacgcgcgg gcttgagata gtcaggcgtg actggagcga gatagccaag     1860
   gagactcagg cgagggttct tgaggccctt ctcaaggacg gtaacgttga ggaggccgta     1920
   agcatagtca aagaagtgac ggagaagctg agcaagtacg aggttccgcc ggagaagctc     1980
   gttatccacg agcagataac gcgcgagctg aaggactaca aggcaacggg cccgcacgtg     2040
   gcgatagcga agaggttagc cgcgaggggc gtcaaaatcc gccccgggac ggtcatcagc     2100
   tacatagtcc tcaagggctc tggaaggata ggcgacaggg cgattccctt cgacgagttc     2160
   gacccggcca agcaccgcta cgacgctgaa tactacatcg agaaccaggt tctgccggcc     2220
   gttgagagga ttctaaaggc cttcggctat agaaaggagg atctgcgcta ccagaagacg     2280
   aggcaggttg ggcttggagc gtggttaaag ccgaagggga agaagggatc ctaa           2334
<210> 50
   <211> 40
   <212> DNA
   <213> Artificial
<220>
   <223> Primer sequence
<400> 50
   gaattcggat ccggaacaca catggcgtgt gagaagcctg 40
<210> 51
   <211> 30
   <212> DNA
   <213> Artificial
<220>
   <223> Primer sequence
<400> 51
   gaattcgtcg acttagctgc agattgggta 30
<210> 52
   <211> 210
   <212> DNA
   <213> Artificial
<220>
   <223> Cloned construct
<400> 52
   ggatccggaa cacacatggc gtgtgagaag cctgttaagg ttcgtgaccc tactactggt       60
   aaggaggtag agctggtacc aatcaaggtg tggcagctag cacccagggg taggaagggc      120
   gtcaagatag gcctattcaa gagccccgaa acaggcaagt acttcagagc caaggtacca      180
   gacgactacc caatctgcag ctaagtcgac                                       210

## Description

### Field of Invention

The present invention relates to novel polypeptides having DNA polymerase activity, and their uses.

### Background

DNA polymerases are enzymes involved *in vivo* in DNA repair and replication, but have become an important *in vitro* diagnostic and analytical tool for the molecular biologist. The enzymes are divided into three main families, based on function and conserved amino acid sequences (see Joyce & Steitz, 1994, Ann. Rev. Biochem. 63: 777-822). In prokaryotes, the main types of DNA polymerases are DNA polymerase I, II and III. DNA polymerase I (encoded by the gene "polA" in *E. coli*) is considered to be a repair enzyme and has 5'-3' polymerase activity and often 3'-5' exonuclease proofreading activity and/or 5'-3' exonuclease activity which when present mediates nick translation during DNA repair. DNA polymerase II (encoded by the gene "polB" in *E. coli*) appears to facilitate DNA synthesis starting from a damaged template strand and thus preserves mutations. DNA polymerase III (encoded by the gene "polC" in *E. coli*) is the replication enzyme of the cell, synthesising nucleotides at a high rate (such as about 30,000 nucleotides per minute) and having no 5'- 3' exonuclease activity.

Other properties of DNA polymerases are derived from their source of origin. For example, several DNA polymerases obtained from thermophilic bacteria have been found to be thermostable, retaining polymerase activity at between 45°C to 100°C, depending on the polymerase. Thermostable DNA polymerases have found wide use in methods for amplifying nucleic acid sequences by thermocycling amplification reactions such as the polymerase chain reaction (PCR) or by isothermal amplification reactions such as strand displacement amplification (SDA), nucleic acid sequence-based amplification (NASBA), self-sustained sequence replication (3SR), and loop-mediated isothermal amplification (LAMP).

The different properties of thermostable DNA polymerases, such as level of thermostability, strand displacement activity, fidelity (error rate) and binding affinity to template DNA and/or RNA and/or free nucleotides, make them suited to different types of amplification reaction. For example, thermostable (typically at temperatures up to 94°C), high-fidelity (typically with 3'-5' exonuclease proof-reading activity), processive and rapidly synthesising DNA polymerases are preferred for PCR. Enzymes which do not discriminate significantly between dideoxy and deoxy nucleotides may be preferred for sequencing. Meanwhile, isothermal amplification reactions require a DNA polymerase with strong strand displacement activity.

The proof-reading DNA polymerases currently available commercially for PCR are derived from species within either the *Pyrococcus* genus or the *Thermococcus* genus of hyperthermophilic euryarchaeota. *Archaea* are a third domain of living organisms, distinct from *Bacteria* and *Eucarya.* These organisms have been isolated predominantly from deep-sea hydrothermal vents ("black smokers") and typically have optimal growth temperatures around 85-99°C. Examples of key species from which proof-reading DNA polymerases for use in PCR have been isolated include *Thermococcus barossii*, *Thermococcus litoralis*, *Thermococcus gorgonarius, Thermococcus pacificus*, *Thermococcus zilligii*, *Thermococcus 9N7, Thermococcus fumicolans*, *Thermococcus aggregans (TY), Thermococcus peptonophilus, Pyrococcus furiosus*, *Pyrococcus sp.* and *Thermococcus KOD.* Takagi et al. (Appl. Env. Microbiol. (1997) 63: 4504-4510) and EP-A-0745675 provide characterisation of the DNA polymerase found in *Pyrococcus* sp. Strain KOD1. This strain has an optimum growth temperature of 95°C.

The commercially available proof-reading DNA polymerases noted above are DNA polymerase II-like, have the basic structure comprising a 3'-5' exonuclease domain followed by a polymerase domain, and a molecular weight of around 85-90kDa. An unusual characteristic of these enzymes is that they often, but not always, have one or more inteins, part or all of which is spliced out *in vivo* to form the mature polymerase. Inteins are genetic elements that disrupt the coding sequence of the genes but in contrast to introns, inteins are transcribed and translated together with the protein. Most inteins comprise two domains, one of which is involved in autocatalytic splicing, and the other of which is a small endonuclease involved in the spread of inteins.

EP 1757698 discloses a DNA polymerase from a *Thermococcus* species.

### Summary of Invention

The present invention provides in one aspect a novel thermostable DNA polymerase for use in reactions requiring DNA polymerase activity such as nucleic acid amplification reactions. The polymerase has been isolated from a new genus of hyperthermophilic euryarchaeota, the *Palaeococcus* genus, which represents a deep-branching lineage of the order Thermococcales that diverged before *Thermococcus* and *Pyrococcus.* The polymerase is suitable for use in thermocycling amplification reactions, even though the optimum growth temperature for the organism is only 83°C (see below).

According to one aspect of the present invention there is provided a polypeptide having thermostable DNA polymerase activity and comprising or consisting essentially of an amino acid sequence with at least 94% identity, for example at least 95%, 96%, 97%, 98% or 99% identity, to *Palaeococcus ferrophilus* DNA polymerase shown in SEQ ID NO:1. Preferably, the polypeptide is isolated.

The *P. ferrophilus* DNA polymerase has the following amino acid sequence:

The predicted molecular weight of this 775 amino acid residue *P. ferrophillus* DNA polymerase shown in SEQ ID NO:1 is about 89,960 Daltons.

The above percentage sequence identity may be determined using the BLASTP computer program with SEQ ID NO:1 as the base sequence. This means that SEQ ID NO:1 is the sequence against which the percentage identity is determined. The BLAST software is publicly available at http://blast.ncbi.nlm.nih.gov/Blast.cgi (accessible on 12 March 2009).

For example, the polypeptide may comprise or consist essentially of any contiguous 698 amino acid sequence included within SEQ ID NO:1. Alternatively or additionally, the polypeptide may by about 775 amino acids in length, for example, from about 750 to 1400 amino acids, or 750 to 1310 amino acids, or 750 to 1305 amino acids, or 775 to 1305 amino acids, or 750 to 1300 amino acids, or 760 to 1300 amino acids, or 770 to 1300 amino acids, or 775 to 1300 amino acids.

The polypeptide may comprise or consist essentially of the amino acid sequence SEQ ID NO:1, or of the amino acid sequence of SEQ ID NO:1 with 1, 2, 3, 4, 5, 6, 7, 8, 9, 10, 11, 12, 13, 14, 15, about 20, about 30, about 40, about 50, about 100, about 200, about 300, about 400, about 500, about 510, 520, 521, 522, 523, 524, 525, 526, 527, 528, 529 or 530 contiguous amino acids added to or removed from any part of the polypeptide and/or 1, 2, 3, 4, 5, 6, 7, 8, 9, 10, 11, 12, 13, 14, 15, about 20, about 30, about 40 or about 50 amino acids or contiguous amino acids added to or removed from the N-terminus region and/or the C-terminus region.

*Palaeococcus ferrophilus* is a barophilic, hyperthermophilic archaeon isolated from a deep-sea hydrothermal vent chimney, and has a reported temperature range for growth of 60-88°C and an optimum growth temperature of 83°C (see Takai et al., 2000, Int. J. Syst. Evol. Microbiol. 50: 489-500). This organism was reported to be the first member of the *Palaeococcus* genus of hyperthermophilic euryarchaeota, and to date there are no known published reports of the identification and characterisation of a DNA polymerase from this genus. Genomic DNA (gDNA) from *P. ferrophilus* has been isolated by the inventors, who used a sophisticated gene walking technique to clone a DNA polymerase, considered to be a DNA polymerase II encoded by a DNA polymerase II (polB) gene.

DNA polymerase II enzymes comprise certain conserved motifs, for example, as described in Kim et al., (2007) J. Microbiol. Biotechnol. 17 1090-1097. Therefore, in a preferred embodiment, the peptide according to the invention comprises one or more of the amino acid sequences:
EX₁X₂X₁₈IKX₃FLX₁₉X₄X₂₀X₁EKDPDX₄X₅X₄TY (SEQ ID NO:36)
GX₆VKEPEX₁GLWX₂X₂₁X₅X₂₂X₈LDX₆X₁X₉LYPSIIX₄THNVSPDT (SEQ ID NO:37)
GFIPSX₅LX₁₀X₁₁LX₅X₂X₂₃RQX₁₂X₄KX₁₃KMK (SEQ ID NO:38)
DYRQX₁AX₅KX₅LANSX₆YGYX₂₄GYX₁₄X₁ (SEQ ID NO:39)
DTDGX₁₅X₁₆A (SEQ ID NO:40)
DEEGX₂₅X₄X₁₇TRGLEX₄VRRDWSX₂IAK (SEQ ID NO:41)
where:

| | |
|---|---|
| X₁=K or R | X₁₄=A or P |
| X₂=E or D | X₁₅=F or L |
| X₃=R or A | X₁₆=Y, F or H |
| X₄=V or I | X₁₇=V, T or I |
| X₅=L or I | X₁₈=M or A |
| X₆=Y or F | X₁₉=K, R or H |
| X₇=N or G | X₂₀=V, I or L |
| X₈=Y or S | X₂₁=N, G or S |
| X₉=S or A | X₂₂=V or A |
| X₁₀=G, K or E | X₂₃=E or T |
| X₁₁=N, D, H or E | X₂₄=Y or T |
| X₁₂=K or E | X₂₅=G or H |
| X₁₃=R, K or T | |

For example, the polypeptide my comprise any two, any three, any four or any five amino acid sequences selected from SEQ ID NOs:36-41 or may comprise all of amino acid sequences SEQ ID NOs:36-41. In a preferred embodiment, the peptide according to the invention may comprise one or both of the amino acid sequences:
LYPSIIX₄THNVSPDT (SEQ ID NO:44)
TRGLEX₄VRRDWSX₂IAK (SEQ ID NO:45).

The polypeptide may be suitable for carrying out a thermocycling amplification reaction, such as a polymerase chain reaction (PCR). This characteristic requires sufficient thermostability to withstand the denaturation cycle, normally 95°C.

The polypeptide of the invention may have a half-life at 95°C of about 0.5-10 h, such as about 1-8 h or about 3-6 h or about 1 h. Even though *P. ferrophilus* has a reported growth range of up to only 88°C (see above), the inventors have surprisingly found that even a crude extract of the DNA polymerase II of SEQ ID NO: 1 is stable for at least 4 h at 95°C. In addition, the extension rate of the polypeptide is surprisingly high at around 8kb within one minute (see Examples below), whereas most prior art enzymes achieve around 2kb within 2 minutes.

The polypeptide may have 3'-5' exonuclease proofreading activity.

In some embodiments, the polypeptide may lack 5'-3' exonuclease activity.

The polypeptide of the invention may be an isolated thermostable DNA polymerase obtainable from *Palaeococcus ferrophilus* and having a molecular weight of about 90,000 Daltons, or about 89,000 - 91,000 Daltons, or an enzymatically active fragment thereof.

The polypeptide according to the invention may comprise or consist essentially of an amino acid sequence with at least 81% identity, for example at least 82%, 83%, 85%, 90%, 95%, 96%, 97%, 98% or 99% identity, to *Palaeococcus ferrophilus* DNA polymerase intein protein shown in SEQ ID NO:2. Preferably the polypeptide is isolated.

The *P. ferrophilus* DNA polymerase intein protein of SEQ ID NO: 2 is a "precursor" protein which includes a single intein region that is spliced out to form the DNA polymerase of SEQ ID NO:1. The precursor protein has the following amino acid sequence:

The intein region which is spliced out of the intein protein of SEQ ID NO:2 to form the DNA polymerase of SEQ ID NO:1 is underlined above. This intein region and variants thereof also form an aspect of the invention.

Therefore, the polypeptide of the invention may comprise the amino acid sequence RQRAIKILANSYYGYYGYAR (SEQ ID NO:35), representing the sequences of the polymerase which flank the intein and which are spliced after excision of the intein. The polypeptide may comprise a sequence having 1, 2, 3, 4, 5, 6, 7, 8, 9 or 10 contiguous amino acids added to or removed from any part of SEQ ID NO:35 and/or 1, 2, 3, 4, 5, 6, 7, 8 or 9 amino acids added to or removed from the N-terminal region or C-terminal region of SEQ ID NO:35. For example, the polypeptide may comprise any portion of SEQ ID NO:35 which itself comprises the "NS" motif or pair of amino acids, representing the splice site.

The predicted molecular weight of the 1305 amino acid residue *P. ferrophilus* DNA polymerase intein protein shown in SEQ ID NO:2 is about 151,550 Daltons.

The polypeptide of the invention may be an isolated thermostable DNA polymerase obtainable from *Palaeococcus ferrophilus* and having a molecular weight of about 152,000 Daltons, or about 151,000 - 153,000 Daltons, or an enzymatically active fragment thereof. The term "enzymatically active fragment" means a fragment of such a polymerase obtainable from *P. ferrophilus* and having enzyme activity which is at least 60%, preferably at least 70%, more preferably at least 80%, yet more preferably 90%, 95%, 96%, 97%, 98%, 99% or 100% that of the full length polymerase being compared to. The given activity may be determined by any standard measure, for example, the number of bases of nucleotides of the template sequence which can be replicated in a given time period. The skilled person is routinely able to determine such properties and activities.

The above percentage sequence identity may be determined using the BLASTP computer program with SEQ ID NO:2 as the base sequence. This means that SEQ ID NO:2 is the sequence against which the percentage identity is determined.

The polypeptide may comprise or consist essentially of the amino acid sequence SEQ ID NO:2, or of the amino acid sequence of SEQ ID NO:2 with 1, 2, 3, 4, 5, 6, 7, 8, 9, 10, 11, 12, 13, 14, 15, about 20, about 30, about 40, about 50, about 100, about 200, about 300, about 400, about 500, about 510, 520, 521, 522, 523, 524, 525, 526, 527, 528, 529 or 530 contiguous amino acids added to or removed from any part of the polypeptide and/or 1, 2, 3, 4, 5, 6, 7, 8, 9, 10, 11, 12, 13, 14, 15, about 20, about 30, about 40 or about 50 amino acids added to or removed from the N-terminus region and/or the C-terminus region.

The polypeptide of the invention may be suitable for use in one or more reactions requiring DNA polymerase activity, for example one or more of the group consisting of: nick translation, second-strand cDNA synthesis in cDNA cloning, DNA sequencing, and thermocycling amplification reactions such as PCR.

In a further aspect of the invention the polypeptide exhibits high fidelity polymerase activity during a thermocycling amplification reaction (such as PCR). High fidelity may be defined as a PCR error rate of less than 1 nucleotide per 300 x 10⁶ amplified nucleotides, for example less than 1 nucleotide per 250 x 10⁶, 200 x 10⁶, 150 x 10⁶, 100 x 10⁶ or 50 x 10⁶ amplified nucleotides. Alternatively, the error rate of the polypeptides may be in the range 1-300 nucleotides per 10⁶ amplified nucleotides, for example 1-200, 1-100, 100-300, 200-300, 100-200 or 75-200 nucleotides per 10⁶ amplified nucleotides. Error rate may be determined using the opal reversion assay as described by Kunkel et al. (1987, Proc. Natl. Acad. Sci. USA 84: 4865-4869).

The polypeptide of the invention may comprise additional functional and structural domain, for example, an affinity purification tag (such as an His purification tag), or DNA polymerase activity-enhancing domains such as the proliferating cell nuclear antigen homologue from *Archaeoglobus fulgidus*, T3 DNA polymerase thioredoxin binding domain, DNA binding protein Sso7d from *Sulfolobus solfataricus*, Sso7d-like proteins, or mutants thereof, or helix-hairpin-helix motifs derived from DNA topoisomerase V.

The DNA polymerase activity-enhancing domain may also be a Cren7 enhancer domain or variant thereof, as defined and exemplified in co-pending International patent application no. PCT/GB2009/000063, which discloses that this highly conserved protein domain from Crenarchael organisms is useful to enhance the properties of a DNA polymerase. International patent application no. PCT/GB2009/000063 is incorporated herein by reference in its entirety.

An example of such a polypeptide has the following 842 amino acid sequence:

In another aspect of the invention there is provided a composition comprising the polypeptide as described herein. The composition may for example include a buffer, and/or most or all ingredients for performing a reaction (such as a DNA amplification reaction for example PCR), and/or a stabiliser (such as *E. coli* GroEL protein, to enhance thermostability), and/or other compounds. The composition is in one aspect enzymatically thermostable.

The invention further provides an isolated nucleic acid encoding the polypeptide with identity to *P. ferrophilus* DNA polymerase. The nucleic acid may, for example, have a sequence as shown below (5'-3'):

The nucleotide of SEQ ID NO:3 encodes the *P. ferrophilus* DNA polymerase of SEQ ID NO: 1 as follows:

The underlined codon "ctc" coding for Leucine in SEQ NO:3 above is a minor tRNA in *E. coli* and, therefore, this codon was changed to "ctg" by the inventors for expression clone work (see Henaut and Danchin (1996) in Escherichia coli and Salmonella typhimurium Cellular and Molecular Biology Vol. 2, 2047-2066, American Society for Microbiology, Washington, DC). The isolated nucleic acid having this amended nucleotide sequence is also encompassed by the invention. The altered codon does not result in any change in the expressed amino acid sequence which is also, therefore, SEQ ID NO:1.

The invention further provides an isolated nucleic acid encoding the polypeptide with identity to the *P. ferrophilus* DNA polymerase intein protein. The nucleic acid may, for example, have a sequence as shown below (5'-3'):

The nucleotide of SEQ ID NO: 4 encodes the P. *ferrophilus* DNA polymerase intein protein of SEQ ID NO: 2 as follows:

The underlined codon "ctc" coding for Leucine in SEQ ID NO:4 above is a minor tRNA in *E. coli* and, therefore, this codon was changed to "ctg" by the inventors as outlined above. The isolated nucleic acid having this amended nucleotide sequence is also encompassed by the invention. The altered codon does not result in any change in the expressed amino acid sequence which is also, therefore, SEQ ID NO:2.

The invention further provides an isolated nucleic acid sequence encoding the fusion protein having amino acid sequence SEQ ID NO:46. The nucleic acid may, for example, have a sequence as shown below (5'-3'):

The nucleic acid sequence SEQ ID NO:47 encodes the amino acid sequence SEQ ID NO:46 as follows:

The underlined codon "ctc" coding for Leucine in SEQ ID NO:47 above is a minor tRNA in *E. coli* and, therefore, this codon was changed to "ctg" by the inventors as outlined above. The isolated nucleic acid having this amended nucleotide sequence is also encompassed by the invention. The altered codon does not result in any change in the expressed amino acid sequence which is also, therefore, SEQ ID NO:46.

Also encompassed by the invention are further variants of the nucleic acids, as defined below.

Further provided is a vector comprising the isolated nucleic acid as described herein.

Additionally provided is a host cell transformed with the vector of the invention.

Also provided is a method for of producing a DNA polymerase of the invention comprising culturing the host cell defined herein under conditions suitable for expression of the DNA polymerase.

A recombinant polypeptide expressed from the host cell is also encompassed by the invention.

In another aspect of the invention there is provided a kit comprising the polypeptide as described herein, and/or the composition as described herein, and/or the isolated nucleic acid as described herein, and/or the vector as described herein, and/or the host cell as described herein, together with packaging materials therefor. The kit may, for example, comprise components including the polypeptide for carrying out a reaction requiring DNA polymerase activity, such as PCR.

The invention further provides a method of amplifying a sequence of a target nucleic acid using a thermocycling reaction, for example PCR, comprising the steps of:
(1) contacting the target nucleic acid with the polypeptide having thermostable DNA polymerase activity or the composition as described herein; and
(2) incubating the target nucleic acid with the polypeptide or the composition under thermocycling reaction conditions which allow amplification of the target nucleic acid.

The present invention also encompasses variants of the polypeptide and intein region as defined herein. As used herein, a "variant" means a polypeptide in which the amino acid sequence differs from the base sequence from which it is derived in that one or more amino acids within the sequence are substituted for other amino acids. Amino acid substitutions may be regarded as "conservative" where an amino acid is replaced with a different amino acid with broadly similar properties. Non-conservative substitutions are where amino acids are replaced with amino acids of a different type.

By "conservative substitution" is meant the substitution of an amino acid by another amino acid of the same class, in which the classes are defined as follows:

| Class | Amino acid examples |
|---|---|
| Nonpolar: | A, V, L, I, P, M, F, W |
| Uncharged polar: | G, S, T, C, Y, N, Q |
| Acidic: | D, E |
| Basic: | K, R, H. |

As is well known to those skilled in the art, altering the primary structure of a peptide by a conservative substitution may not significantly alter the activity of that peptide because the side-chain of the amino acid which is inserted into the sequence may be able to form similar bonds and contacts as the side chain of the amino acid which has been substituted out. This is so even when the substitution is in a region which is critical in determining the peptide's conformation.

Non-conservative substitutions are possible provided that these do not interrupt with the function of the DNA binding domain polypeptides.

Broadly speaking, fewer non-conservative substitutions will be possible without altering the biological activity of the polypeptides.

Determination of the effect of any substitution (and, indeed, of any amino acid deletion or insertion) is wholly within the routine capabilities of the skilled person, who can readily determine whether a variant polypeptide retains the thermostable DNA polymerase activity according to the invention. For example, when determining whether a variant of the polypeptide falls within the scope of the invention, the skilled person will determine whether the variant retains enzyme activity (i.e., polymerase activity) at least 60%, preferably at least 70%, more preferably at least 80%, yet more preferably 90%, 95%, 96%, 97%, 98%, 99% or 100% of the non-variant polypeptide. Activity may be measured by, for example, any standard measure such as the number of bases of a template sequence which can be replicated in a given time period.

Variants of the polypeptide and/or intein region may comprise or consist essentially of an amino acid sequence with at least 90% identity, for example at least 91%, 92%, 93%, 94%, 95%, 96%, 97%, 98% or 99% identity to SEQ ID NO:1 and/or 81% identity, for example at least 82%, 83%, 85%, 90%, 95%, 96%, 97%, 98% or 99% identity to SEQ ID NO:2.

Using the standard genetic code, further nucleic acids encoding the polypeptides may readily be conceived and manufactured by the skilled person. The nucleic acid may be DNA or RNA and, where it is a DNA molecule, it may for example comprise a cDNA or genomic DNA.

The invention encompasses variant nucleic acids encoding the polypeptide of the invention. The term "variant" in relation to a nucleic acid sequences means any substitution of, variation of, modification of, replacement of, deletion of, or addition of one or more nucleic acid(s) from or to a polynucleotide sequence providing the resultant polypeptide sequence encoded by the polynucleotide exhibits at least the same properties as the polypeptide encoded by the basic sequence. The term therefore includes allelic variants and also includes a polynucleotide which substantially hybridises to the polynucleotide sequence of the present invention. Such hybridisation may occur at or between low and high stringency conditions. In general terms, low stringency conditions can be defined a hybridisation in which the washing step takes place in a 0.330-0.825 M NaCl buffer solution at a temperature of about 40-48°C below the calculated or actual melting temperature (Tₘ) of the probe sequence (for example, about ambient laboratory temperature to about 55°C), while high stringency conditions involve a wash in a 0.0165-0.0330 M NaCl buffer solution at a temperature of about 5-10°C below the calculated or actual Tₘ of the probe(for example, about 65°C). The buffer solution may, for example, be SSC buffer (0.15M NaCl and 0.015M tri-sodium citrate), with the low stringency wash taking place in 3 x SSC buffer and the high stringency wash taking place in 0.1 x SSC buffer. Steps involved in hybridisation of nucleic acid sequences have been described for example in Sambrook et al. (1989; Molecular Cloning, Cold Spring Harbor Laboratory Press, Cold Spring Harbor).

Typically, variants have 85% or more of the nucleotides in common with the nucleic acid sequence of the present invention, for example 86%, 87%, 88%, 89%, 90%, 91%, 92%, 93%, 94%, 95%, 96%, 97%, 98% or 99% or greater sequence identity.

Variant nucleic acids of the invention may be codon-optimised for expression in a particular host cell.

DNA polymerases and nucleic acids of the invention may be prepared synthetically using conventional synthesisers. Alternatively, they may be produced using recombinant DNA technology or isolated from natural sources followed by any chemical modification, if required. In these cases, a nucleic acid encoding the chimeric protein is incorporated into suitable expression vector, which is then used to transform a suitable host cell, such as a prokaryotic cell such as *E. coli.* The transformed host cells are cultured and the protein isolated therefrom. Vectors, cells and methods of this type form further aspects of the present invention.

Sequence identity between nucleotide and amino acid sequences can be determined by comparing an alignment of the sequences. When an equivalent position in the compared sequences is occupied by the same amino acid or base, then the molecules are identical at that position. Scoring an alignment as a percentage of identity is a function of the number of identical amino acids or bases at positions shared by the compared sequences. When comparing sequences, optimal alignments may require gaps to be introduced into one or more of the sequences to take into consideration possible insertions and deletions in the sequences. Sequence comparison methods may employ gap penalties so that, for the same number of identical molecules in sequences being compared, a sequence alignment with as few gaps as possible, reflecting higher relatedness between the two compared sequences, will achieve a higher score than one with many gaps. Calculation of maximum percent identity involves the production of an optimal alignment, taking into consideration gap penalties.

In addition to the BLASTP computer program mentioned above, further suitable computer programs for carrying out sequence comparisons are widely available in the commercial and public sector. Examples include the MatGat program (Campanella et al., 2003, BMC Bioinformatics 4: 29), the Gap program (Needleman & Wunsch, 1970, J. Mol. Biol. 48: 443-453) and the FASTA program (Altschul et al., 1990, J. Mol. Biol. 215: 403-410). MatGAT v2.03 is freely available from the website "http://bitincka.com/ledion/matgat/" (accessed 12 March 2009) and has also been submitted for public distribution to the Indiana University Biology Archive (IUBIO Archive). Gap and FASTA are available as part of the Accelrys GCG Package Version 11.1 (Accelrys, Cambridge, UK), formerly known as the GCG Wisconsin Package. The FASTA program can alternatively be accessed publicly from the European Bioinformatics Institute (http://www.ebi.ac.uk/fasta, accessed 12 March 2009) and the University of Virginia (http://fasta.biotech.virginia.edu/fasta_www/cgi or http://fasta.bioch.virginia.edu/fasta_www2/fasta_list2.shtml, accessed 12 March 2009). FASTA may be used to search a sequence database with a given sequence or to compare two given sequences (see http://fasta.bioch.virginia.edu/fasta_www/cgi/ search_frm2.cgi, accessed 12 March 2009). Typically, default parameters set by the computer programs should be used when comparing sequences. The default parameters may change depending on the type and length of sequences being compared. A sequence comparison using the MatGAT program may use default parameters of Scoring Matrix = Blosum50, First Gap = 16, Extending Gap = 4 for DNA, and Scoring Matrix = Blosum50, First Gap = 12, Extending Gap = 2 for protein. A comparison using the FASTA program may use default parameters of Ktup = 2, Scoring matrix = Blosum50, gap = -10 and ext = -2.

In one aspect of the invention, sequence identity is determined using the MatGAT program v2.03 using default parameters as noted above.

As used herein, a "DNA polymerase" refers to any enzyme that catalyzes polynucleotide synthesis by addition of nucleotide units to a nucleotide chain using a nucleic acid such as DNA as a template. The term includes any variants and recombinant functional derivatives of naturally occurring nucleic acid polymerases, whether derived by genetic modification or chemical modification or other methods known in the art.

As used herein, "thermostable" DNA polymerase activity means DNA polymerase activity which is relatively stable to heat and functions at high temperatures, for example 45-100°C, preferably 55-100°C, 65-100°C, 75-100°C, 85-100°C or 95-100°C, as compared, for example, to a non-thermostable form of DNA polymerase.

### Brief Description of Figures

Particular non-limiting embodiments of the present invention will now be described with reference to the following Figures, in which:

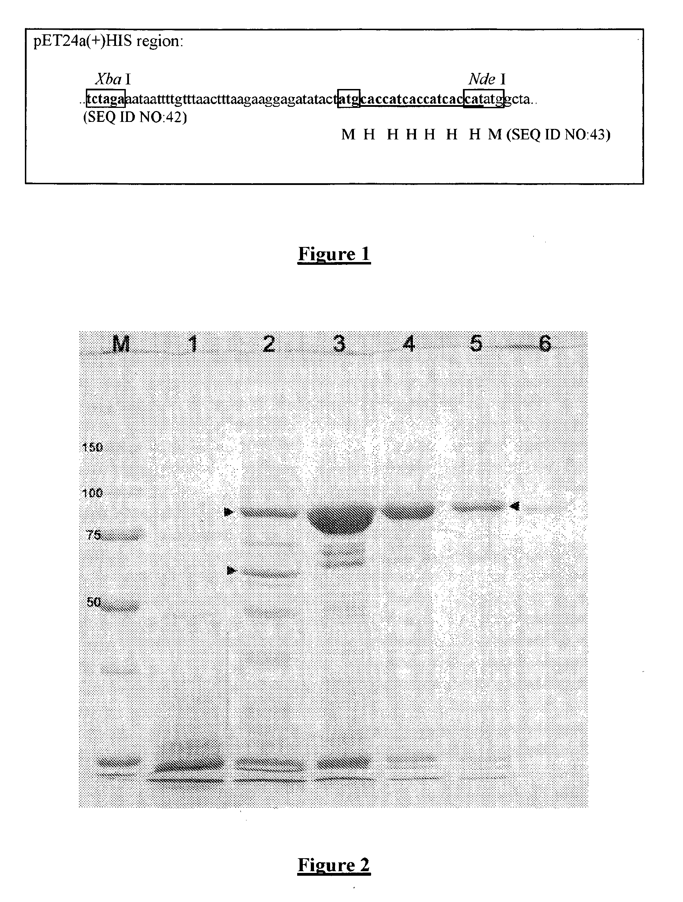

Figure 1 is a diagram showing the structure of the pET24a(+)HIS region used in cloning of a *Palaeococcus ferrophilus* DNA polymerase and DNA polymerase intein protein according to a first and second embodiment of the invention, respectively;
Figure 2 is an SDS PAGE gel of fractionated expressed *Palaeococcus ferrophilus* DNA polymerase and DNA polymerase intein protein according to the first and second embodiment of the invention referred to in Figure 1. Lane M is a Bio-Rad Precision Plus Protein Standard; lane 1 is induced negative control (equivalent of 100µl *E.coli*); lane 2 is induced HIS-tagged *P. ferrophilus* DNA polymerase intein protein (equivalent of 100µl *E.coli*), with the upper arrow shows HIS-tagged DNA polymerase and lower arrow putative self-excised intein region; lane 3 is induced HIS-tagged *P. ferrophilus* DNA polymerase (equivalent of 50µl *Ecoli*); lane 4 is induced HIS-tagged *P. ferrophilus* DNA polymerase (equivalent of 12.5µl *E.coli*); lane 5 is induced HIS-tagged *P. ferrophilus* DNA polymerase (equivalent of 5µl *E.coli*); and lane 6 is 25u Pfu polymerase;

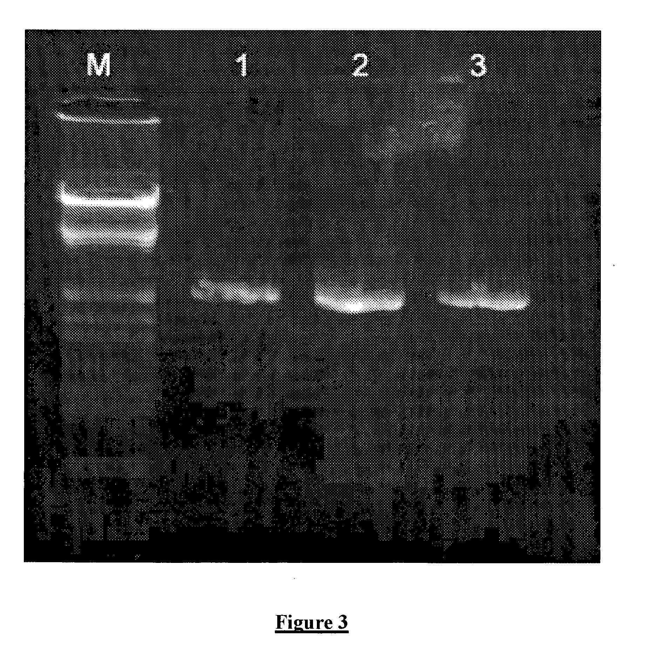

Figure 3 is an agarose gel of fractionated PCR reaction samples following amplification of lambda (λ) DNA using the *Palaeococcus ferrophilus* DNA polymerase and DNA polymerase intein protein according to the first and second embodiments of the invention referred to in Figures 1 and 2. Lane M is an *Eco*R I/*Hin*d III Lambda DNA marker (band sizes (in bp):564, 831, 947, 1375, 1584, 1904, 2027, 3530, 4268, 4973, 5148, 21226); lane 1 is a PCR sample amplified using 1.25u Pfu polymerase (positive control); lane 2 is a PCR sample amplified using 0.025µl of *E. coli* extract of *P. ferrophilus* DNA polymerase; and lane 3 is a PCR sample amplified using 0.025µl of *E. coli* extract of *P. ferrophilus* DNA polymerase intein protein; and

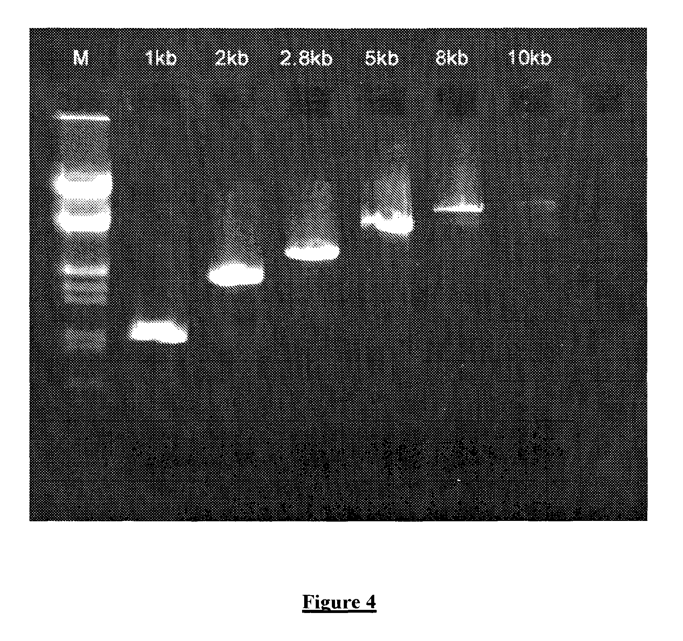

Figure 4 is an agarose gel of PCR reaction samples following amplification of various lengths of DNA template using *P. ferrophilus* DNA polymerase. Lane M is an *Eco*R I/*Hin*d III Lambda DNA marker as above; the remaining lanes show PCR products of size 1kb, 2kb, 2.8kb, 5kb, 8kb and 10kb, respectively.

### Examples

Lyophilized cultures of *Palaeococcus ferrophilus* were obtained from the Deutsche Sammlung von Mikroorganismen und Zellkulturen GmbH (German Collection of Microorganisms and Cell Cultures; Accession No. DSM 13482). As described below, following extraction and amplification of gDNA from the cultures, a gene walking method was used, as outlined below, to reach the predicted 5' start and the 3' stop of a putative DNA polymerase B gene ("DNA polB") encoding a putative DNA polymerase II.

### Example 1 Genomic DNA extraction

The method for genomic DNA extraction from *P. ferrophilus* cultures was derived from Gotz et al. (2002; Int. J. Syst. Evol. Microbiol. 52: 1349-1359) which is a modification of a method described in Ausubel et al. (1994; Current Protocols in Molecular Biology, Wiley, New York).

Cell pellets were resuspended in 567ul 1x TE buffer (10mM Tris/HCl, pH8.0; 1mM EDTA), 7.5% Chelex 100 (Sigma), 50mM EDTA (pH7.0), 1% (w/v) SDS and 200µg Proteinase K and incubated with slow rotation for 1h at 50°C. Chelex was removed by centrifugation. Then 100µl 5M NaCl and 80µl 10% (w/v) cetyltrimethylammonium bromide in 0.7M NaCl were added to the cell lysate and the sample incubated for 30 mins at 65°C. The DNA was extracted with phenol/chloroform, isopropanol precipitated and the DNA resuspended in water. DNA concentration was estimated on a 1% agarose gel.

### Example 2 Initial screening for putative DNA polB gene

The screening method was derived from Shandilya et al. (2004, Extremophiles 8: 243-251) and Griffiths et al. (2007, Protein Expression & Purification 52:19-30).

Using degenerate Pol primers ARCHPOLR1 and ARCHPOLF1 (see below), a -730bp fragment was amplified from 10ng *P. ferrophilus* gDNA.

The ARCHPOLR1 primer has the sequence:
5' - CGC GGG AGA ACC TGG TTN TCD ATR TAR TA - 3' (SEQ ID NO:5)
   (corresponding to the amino acid sequence YYIENQVLP (SEQ ID NO:6)); and
the ARCHPOLF1 primer has the sequence:
5' - TAC TAC GGA TAG GCC AAR GCN AGR TGG TA - 3' (SEQ ID NO:7)
   (corresponding to the amino acid sequence YYGXAKARW (SEQ ID NO:8)).

"X" in SEQ ID NO:8 represents a "STOP" codon, as derived from the primer sequence which is as used by Griffiths *et al.* The primer is still effective in this gene walking method, as demonstrated in the present application and also by the work of Griffiths *et al.*

The PCR reaction mix was as follows:

| | |
|---|---|
| 10x PCR Buffer (750mM Tris-HCl, pH8.8, 200mM (NH₄)₂SO₄, 0.1% (v/v) Tween-20) | 10µl |
| 5mM dNTPs | 2µl |
| 5' primer (10pM/µl) | 2.5µl |
| 3' primer (10pM/µl) | 2.5µl |
| gDNA | 10ng |
| *Taq* DNA Polymerase (5u/µl) | 0.25µl |
| Water | To 50µl. |

PCR cycling conditions were 4 minute initial denaturation at 94°C followed by 15 cycles of: 10 seconds denaturation at 94°C, 30 seconds annealing at 60°C (reducing by 1°C per cycle), 1 minute extension at 72°C. This was followed by a further step of 35 cycles of: 10 seconds denaturation at 94°C, 10 seconds annealing at 55°C, 1 minute extension at 72°C. Final extension at 72°C for 7 mins. 4°C hold.

A ∼730bp amplified product was TA cloned (Invitrogen pCR2.1 kit. Cat#K2000-01) and sequenced using M13 Forward (5'-TGTAAAACGACGGCCAGT-3' (SEQ ID NO:9)) and Reverse (5'-AGCGGATAACAATTTCACACAGGA-3' (SEQ ID NO:10)) primers on an ABI-3100 DNA sequencer. Sequencing data confirmed the fragment was of a putative PolB gene.

Thereafter, a specific lower primer "13482_L1" was designed and used in PCR with THERMOPOL_F2 to amplify a larger ∼2475bp fragment.

The "13482_L1" primer has the sequence:
5' - TAT CTC GCT CCA GTC ACG CC - 3' (SEQ ID NO:11); and
the THERMOPOL_F2 has the sequence:
5' - AGG GAG TTC TTC CCN ATG GAR GC - 3' (SEQ ID NO:12)
   (corresponding to the amino acid sequence KEFFPMEA (SEQ ID NO:13)).

The PCR reaction mix was as follows:

| | |
|---|---|
| 10x PCR Buffer (750mM Tris-HCl, pH8.8, 200mM (NH₄)₂SO₄, 0.1% (v/v) Tween-20) | 5µl |
| 5mM dNTPs | 2µl |
| 5' primer (10pM/µl) | 2.5µl |
| 3' primer (10pM/µl) | 2.5µl |
| gDNA | 10ng |
| *Taq* DNA Polymerase (5u/µl) | 0.25µl |
| Water | To 50µl. |

PCR cycling conditions were 4 minute initial denaturation at 94°C followed by 15 cycles of: 10 seconds denaturation at 94°C, 10 seconds annealing at 60°C (reducing by 1°C per cycle), 2 minute extension at 72°C. This was followed by a further step of 35 cycles of: 10 seconds denaturation at 94°C, 10 seconds annealing at 55°C, 2 minute extension at 72°C. Final extension was at 72°C for 7mins. 4°C hold.

A ~2475bp amplified product was ExoSAP treated and sequenced using primer 13482_L1, and later 13482_L2 (5'-AACACCGCAAAGGCTCTCA-3' (SEQ ID NO:14)) and 13482_L3 (5'-GCTTGAGTTCATCCCCCGT-3' (SEQ ID NO:15)).

### Example 3 Gene walking

From the amplification product obtained in Example 2, primers were designed to 'walk along' *P. ferrophilus* gDNA to reach the 5' start (N-terminus of gene product) and 3' stop (C-terminus of gene product) of the putative DNA po1B gene.

10ng gDNA was digested individually with 5u of various 6 base pair-cutter restriction endonucleases in 10µl reaction volume and incubated for 3 h at 37°C. 12 individual digest reactions were run, using a unique 6-cutter restriction enzyme (RE) for each. 5µl digested template was then self-ligated using 12.5u T4 DNA Ligase, 1µl 10x ligase buffer in 50µl reaction volume, with an overnight incubation at 16°C.

Self-ligated DNA was then used as template in two rounds of PCR, the second of which used nested primers to give specificity to amplification.

### C-terminus:

Primers were designed from the ~730bp sequenced fragment to 'walk' to the C-terminal end of the DNA polymerase gene product.

The primers were:
13482_C-ter_Upper1
   5'-TCAGGCGAGGGTTCTTGAGG - 3' (SEQ ID NO:16)
13482_C-ter_Upper_Nested1
   5'-CGTGGCGATAGCGAAGAGGT-3' (SEQ ID NO:17)
13482_C-ter_Lower1
   5'-TATCTCGCTCCAGTCACGCC-3' (SEQ ID NO:18)

### First round PCR:

The PCR reaction mix was as follows:

| | |
|---|---|
| Self-ligation reaction (∼100pg/µl DNA) | 2µl |
| 10x PCR Buffer (200mM Tris-HCl, pH8.8, 100mM KCl, 100mM (NH₄)₂SO₄, 1% (v/v) Triton X-100, 20mM MgSO₄) | 5µl |
| 5mM dNTPs | 2µl |
| 13482_C-ter_Upper1 | 25pM |
| 13482_C-ter_Lower1 | 25pM |
| Taq/Pfu (20:1) (5u/µl) | 1.25u |
| Water | To 50µl. |

Cycling conditions were 4 minute initial denaturation at 94°C followed by 35 cycles of: 10 seconds denaturation at 94°C, 10 seconds annealing at 55°C, 5 minute extension at 72°C. Final extension was at 72°C for 7mins. 4°C hold.

### Second round (nested) PCR:

The PCR reaction mix was as follows:

| | |
|---|---|
| 1^{st} round PCR reaction | 1µl |
| 10x PCR Buffer (200mM Tris-HCl, pH8.8, 100mM KCl, 100mM (NH₄)₂SO₄, 1% (v/v) Triton X-100, 20mM MgSO₄) | 5µl |
| 5mM dNTPs | 2µl |
| 13482_C-ter_Upper_Nested1 | 25pM |
| 13482_C-ter_Lower1 | 25pM |
| Taq/Pfu (20:1) (5u/µl) | 1.25u |
| Water | To 50µl. |

Cycling conditions were 4 minute initial denaturation at 94°C followed by 25 cycles of: 10 seconds denaturation at 94°C, 10 seconds annealing at 55°C, 5 minute extension at 72°C. Final extension was at 72°C for 7mins. 4°C hold.

PCR fragments between ∼0.5kb and ∼2.5kb were obtained from *Nco* I, *Hin*d III, *Nhe* I, *Fsp* I digested/self-ligated reaction templates.

These fragments were sequenced using the nested round primers. Sequencing of fragments indicated that the C-terminal STOP codon of the DNA polymerase gene had been reached.

### N-terminus:

Primers were designed from the ∼2475bp sequenced fragment to 'walk' to the N-terminus of the DNA polymerase gene product.

These primers were:
13482_N-ter_Upper1
   5'-CGCTGGCGGCTACGTGAAGG - 3' (SEQ ID NO:19)
13482_N-ter_Lower1
   5'-TCTCGTCGTACTCCCTTCCG - 3' (SEQ ID NO-20)
13482_N-ter_Lower_Nested1
   5'- GTTTGGGGCCAGTTCGTT -3' (SEQ ID NO:21)

### First round PCR:

The PCR reaction mix was as follows:

| | |
|---|---|
| Self-ligation reaction (∼100pg/µl DNA) | 2µl |
| 10x PCR Buffer | 5µl |

(200mM Tris-HCl, pH8.8, 100mM KCl, 100mM (NH₄)₂SO₄, 1% (v/v) Triton X-100, 20mM MgSO₄)

| | |
|---|---|
| 5mM dNTPs | 2µl |
| 13482_N-ter_Upper1 | 25pM |
| 13482_N-ter_Lower1 | 25pM |
| Taq/Pfu (20:1) (5u/µl) | 1.25u |
| Water | To 50µl. |

Cycling conditions were 4 minute initial denaturation at 94°C followed by 35 cycles of: 10 seconds denaturation at 94°C, 10 seconds annealing at 55°C, 5 minute extension at 72°C. Final extension was at 72°C for 7mins. 4°C hold.

### Second round (nested) PCR:

The PCR reaction mix was as follows:

| | |
|---|---|
| 1^{st} round PCR reaction | 1µl |
| 10x PCR Buffer | 5µl |

(200mM Tris-HCl, pH8.8, 100mM KCl, 100mM (NH₄)₂SO₄, 1% (v/v) Triton X-100, 20mM MgSO₄)

| | |
|---|---|
| 5mM dNTPs | 2µl |
| 13482_N-ter_Upper_1 | 25pM |
| 13482_N-ter_Lower_nested_1 | 25pM |
| Taq/Pfu (20:1) (5u/µl) | 1.25u |
| Water | To 50µl. |

Cycling conditions were 4 minute initial denaturation at 94°C followed by 25 cycles of: 10 seconds denaturation at 94°C, 10 seconds annealing at 55°C, 5 minute extension at 72°C. Final extension was at 72°C for 7mins. 4°C hold.

PCR fragments between -0.5kb and -Ikb were obtained from *Hin*d III, *Apa* I, *BamH* I digested/self-ligated reaction templates.

These fragments were sequenced using the nested round primers to yield new DNA sequence data towards the N-terminus of the DNA polymerase gene product.

Primers were designed from the new sequence data to 'walk' to the start of the N-terminus of the DNA polymerase gene product.

These were:
13482_Nter_Upper2
   5'-AAAGTGAGGCTCGCGTCATC-3' (SEQ ID NO:22)
13482_Nter_Lower2
   5'-ACTCCTCGCCCTCGTGATAG-3' (SEQ ID NO:23)
13482_Nter_Lower_Nested2
   5'-ATTTTCAGCTCCTCGTCCCC-3' (SEQ ID NO:24)

### First round PCR:

The PCR reaction mix was as follows:

| | |
|---|---|
| Self-ligation reaction (∼100pg/µl DNA) | 2µl |
| 10x PCR Buffer | 5µl |

(200mM Tris-HCl, pH8.8, 100mM KCl, 100mM (NH₄)₂SO₄, 1% (v/v) Triton X-100, 20mM MgSO₄)

| | |
|---|---|
| 5mM dNTPs | 2µl |
| 13482_N-ter_Upper2 | 25pM |
| 13482_N-ter_Lower2 | 25pM |
| Taq/Pfu (20:1) (5u/µl) | 1.25u |
| Water | To 50µl. |

Cycling conditions were 4 minute initial denaturation at 94°C followed by 35 cycles of: 10 seconds denaturation at 94°C, 10 seconds annealing at 55°C, 5 minute extension at 72°C. Final extension was at 72°C for 7mins. 4°C hold.

### Second round (nested) PCR:

The PCR reaction mix was as follows:

| | |
|---|---|
| 1^{st} round PCR reaction | 1µl |
| 10x PCR Buffer | 5µl |

(200mM Tris-HCl, pH8.8, 100mM KCl, 100mM (NH₄)₂SO₄, 1% (v/v) Triton X-100, 20mM MgSO₄)

| | |
|---|---|
| 5mM dNTPs | 2µl |
| 13482_N-ter_Upper2 | 25pM |
| 13482_N-ter_Lower Nested2 | 25pM |
| Taq/Pfu (20:1) (5u/µl) | 1.25u |
| Water | To 50µl. |

Cycling conditions were 4 minute initial denaturation at 94°C followed by 25 cycles of: 10 seconds denaturation at 94°C, 10 seconds annealing at 55°C, 5 minute extension at 72°C. Final extension was at 72°C for 7mins. 4°C hold.

PCR fragments between ∼0.5kb and ∼3kb were obtained from *Nco* I, *Nsi* I, *Xho* I digested/self-ligated reaction templates.

These fragments were sequenced using the nested round primers. Sequencing of fragments indicated that the N-terminal ATG start codon of the DNA polymerase gene had been reached.

### Example 4 Amplification of DNA polymerase gene

The gene walking protocols described in Example 3 reached the predicted start and stop of the DNA polymerase gene. Specific primers were designed to amplify a ∼3.9kb fragment incorporating the ~2.3kb DNA polymerase-encoding domain and a ∼1.6kb intein-encoding domain. The start position was determined by alignments with previously reported DNA polymerases (e.g. *Pfu*).

Restriction sites (underlined) were built into primers to allow easy cloning into vectors.

The primers were:
13482_FL_Start_(*Nde*I)
   5'-AAGCTTCATATGATCCTGGATGCTGACTACATAACCGAGAATGG-3' (SEQ ID NO: 25)
13482_STOP_(*Sa*/I)
   5'-GAATTCGTCGACTTACTTCTTCCCCTTCGGCTTTAACCA-3' (SEQ ID NO: 26)

Gene products were amplified using a high fidelity Phusion DNA polymerase (New England Biolabs).

The PCR solution consisted of:

| | |
|---|---|
| 5x HF Phusion reaction Buffer | 20µl |
| 5mM dNTPs | 4µl |
| 5' primer [13482_FL_Start_(*Nde*I)] | 25pM |
| 3' primer [13482_STOP_(*Sal*I)] | 25pM |
| gDNA | 10ng |
| Phusion DNA Polymerase (2u/µl) | 0.5µl |
| Water | To 100µl. |

Cycling conditions were: 30 seconds initial denaturation at 98°C followed by 25 cycles of: 3 seconds denaturation at 98°C, 10 seconds annealing at 55°C, 2.5 minute extension at 72°C. Final extension was at 72°C for 7mins. 4°C hold.

### Example 5 pET24α(+)HIS vector construction

The pET24a(+) vector (Novagen) was modified to add a 6x HIS tag upstream of *Nde*I site (see Figure 1). The HIS tag was inserted between *Xba*I and *Bam*HI sites as follows.

An overlapping primer pair, of which an upper primer (*Xba*I) has the sequence: a lower primer (*BamH*I) has the sequence: were amplified by PCR, RE digested and ligated into pET24a(+). The ligation reaction was transformed into *E. coli* TOP10F' (Invitrogen) and plated on Luria Broth plates plus kanamycin. Colonies were screened by PCR and verified by sequencing using T7 sequencing primers:
T7_Promoter: 5'-AAATTAATACGACTCACTATAGGG-3' (SEQ ID NO:29)
T7_Terminator: 5'-GCTAGTTATTGCTCAGCGG-3' (SEQ ID NO:30)

### Example 6 Cloning of DNA polymerase

The ∼3.9kb fragment PCR product from Example 4 was purified using Promega Wizard purification kit and then RE digested using *Nde* I/*Sal* I. DNA was phenol/chloroform extracted, ethanol-precipitated and resuspended in water. The fragment was then ligated into pET24a(+) and pET24a(+)HIS, between *Nde* I and *Sal* I, and electroporated into KRX (pRARE2)cells (Promega). Colonies were screened by PCR using vector-specific T7 primers. The KRX (pRARE2) cell strain was produced by electroporating the pRARE2 plasmid (isolated from Rosetta2 [EMD Biosciences]) into *E.coli* KRX (Promega). The pRARE2 plasmid supplies tRNAs for seven rare codons (AUA, AGG, AGA, CUA, CCC, CGG, and GGA) on a chloramphenicol-resistant plasmid.

### Example 7 Expression of DNA polymerase

Recombinant colonies from Example 6 were grown up overnight in 5 ml Luria Broth (including Kanamycin/Chloramphenicol). 50 ml Terrific Broth baffled shake flasks were inoculated by 1/100 dilution of overnight culture. Cultures were grown at 37°C, 275 rpm to OD₆₀₀∼1 then brought down to 24°C and induced with L-rhamnose to 0.1% final concentration, and IPTG to 10mM final concentration. Cultures were incubated for a further 18h at 24°C, 275rpm. 10ml of the culture was then harvested by centrifugation for 10mins at 5,000xg and cells were resuspended in 1ml Lysis buffer (50mM Tris-HCl, pH8.0, 100mM NaCl, 1mM EDTA) and sonicated for 2 bursts of 30s (40v) on ice. Samples were centrifuged at 5,000xg for 5min and heat lysed at 70°C for 20min to denature background *E.coli* proteins. Samples were centrifuged and aliquots of supernatant were size fractionated on 8% SDS-PAGE.

As shown in Figure 2, lane 2, expressed protein bands were visible at the expected molecular weight of ∼90kDa (DNA polymerase) and ∼62kDa (intein).

### Example 8 Ampification of DNA polymerase gene (minus intein)

The DNA polymerase sequence either side of the intein were individually PCR amplified and ligated to create a single fragment encoding the full length (∼2.3kb) DNA polymerase gene.

Primers for amplification of DNA pol sequence upstream of intein were:
13482_FL_Start_(*Nde*I) with the sequence
   5'-AAGCTTCATATGATCCTGGATGCTGACTACATAACCGAGAATGG-3' (SEQ ID NO:25)
13482_lower (*Eco*RI) with the sequence
   5'-AAGTTTGAATTCGCCAGGATCTTGATTGCCCGCTGC-3' (SEQ ID NO:31)

Primers to amplify DNA pol sequence downstream of intein were:
13482_ upper(*Eco*R*I*) with the sequence
   5'-CCTGGCGAATTCTTATTACGGCTACTACGGCTACGCAAG-3' (SEQ ID NO:32)
13482_STOP_(*Sal*I) with the sequence
   5'-GAATTCGTCGACTTACTTCTTCCCCTTCGGCTTTAACCA-3' (SEQ ID NO:26)

Gene products were amplified using a high fidelity Phusion DNA polymerase (New England Biolabs).

The PCR solution consisted of:

| | |
|---|---|
| 5x HF Phusion reaction Buffer | 20µl |
| 5mM dNTPs | 4µl |
| 5' primer | 25pM |
| 3' primer | 25pM |
| gDNA | 10ng |
| Phusion DNA Polymerase (2u/µl) | 0.5µl |
| Water | To 100µl. |

Cycling conditions were: 30 seconds initial denaturation at 98°C followed by 25 cycles of: 3 seconds denaturation at 98°C, 10 seconds annealing at 55°C, 1 minute extension at 72°C. Final extension was at 72°C for 7mins. 4°C hold.

The 1473bp and 855bp amplified fragments were visualised by agarose gel electrophoresis.

### Example 9 Cloning of DNA polymerase (minus intein)

PCR products from Example 8 were purified using Promega 'Wizard' purification kit and then RE digested using *NdeI*/*Eco*RI and *Eco*RI/*Sal*I. DNA was phenol/chloroform extracted, EtOH precipitated and resuspended in water. The 1473bp fragment was ligated into pET24a+HIS, and electroporated into KRX (pRARE2) cells. Colonies were screened by PCR using vector-specific T7 primers.

A recombinant colony was grown up in 5ml LB and plasmid mini-prepped using a Macherey Nagel spin column. The plasmid was RE digested with *Eco*RI/*Sal*I and ligated with the 855bp fragment.

The full length DNA polymerase recombinant clones were screened by PCR using 13482_FL_Start*_*(*Nde*I) and 13482_STOP_(*Sal*I) primers.

### Example 10 Expression of DNA polymerase (minus intein)

Positive colonies from Example 9 were grown up overnight in 5ml Luria Broth (including Kanamycin/Chloramphenicol). 50ml Terrific Broth baffled shake flasks were inoculated by 1/100 dilution of o/n culture. Cultures were grown at 37°C, 275rpm to OD₆₀₀∼1 then brought down to 24°C and induced with L-rhamnose to 0.1% final, and IPTG to 10mM final. Cultures were incubated for a further 18hrs at 24°C, 275rpm. 10ml of the culture was then harvested by centrifugation for 10mins at 5,000xg and cells were resuspended in 1ml Lysis buffer (50mM Tris-HCl (pH8.0), 100mM NaCl, 1mM EDTA) and sonicated for 2x 30secs (40v) on ice. Samples were centrifuged at 5,000xg for 5mins and heat lysed at 70°C for 20mins to denature background *E.coli* proteins. Samples were centrifuged and aliquots of supernatant were run out on 8% SDS-PAGE. The cloned DNA polymerase was expressed at -90kDa, as shown in lanes 3-5 of Figure 2.

### Example 11 PCR activity assay

PCR activity of the samples obtained in Example 10 was tested in a 2kb λDNA PCR assay. *Pfu* DNA polymerase (1.25 u) was used as positive control.

The PCR solution contained:

| | |
|---|---|
| 10x PCR Buffer | 5µl |

(200mM Tris-HCl, pH8.8, 100mM KCl, 100mM (NH₄)₂SO₄, 1% (v/v) Triton X-100, 20mM MgSO₄)

| | |
|---|---|
| 5mM dNTP mix | 2µl |
| Enzyme test sample | 1µl |
| Upper λ primer | 50pM |
| Lower λ primer | 50pM |
| λDNA | 10ng |
| Water | To 50µl. |

The Upper λ primer has the sequence:
5' - CCTGCTCTGCCGCTTCACGC - 3' (SEQ ID NO:33)
while the Lower λ primer has the sequence:
5' - CCATGATTCAGTGTGCCCGTCTGG- 3' (SEQ ID NO:34)

PCR proceeded with 35 cycles of: 3 seconds denaturation at 94°C, 10 seconds annealing at 55°C, 2 minutes extension at 72°C. Final extension at 72°C for 7 mins. 4°C hold.

Aliquots of the reaction products were run out on a 1% agarose gel, and the *P. ferrophilus* DNA polymerase and DNA polymerase intein protein (which self-excises to form a DNA polymerase and intein region) were found to amplify the expected 2 kb λ DNA fragment as shown in Figure 3.

In addition, the *P. ferrophilus* DNA polymerase was used in a PCR reaction as above but with an extension time of only 1 minute. Figure 4 shows that the polymerase was, surprisingly, capable of amplifying a product of up to 8kb in this short extension time period, suggesting high processivity.

### Example 12 Thermostability assay

Thermostability of *P. ferrophilus* DNA polymerase (without intein) was tested using the 2kb λDNA PCR assay as described above in Example 11. Crude extract samples of the DNA polymerase were incubated at 95°C for up to 180 min, then used in the 2kb λDNA PCR assay. Under the conditions used, the DNA polymerase was found to have a half-life of approximately 60 min (data not shown).

This example demonstrates that the *P. ferrophilus* DNA polymerase was thermostable and thus highly suitable for thermocycling reactions such as PCR.

### Example 13 Preparation of fusion protein of P. ferrophilus DNA polymerase with Cren7 domain

A BamHI restriction enzyme site was introduced before the TAA (stop) codon of *P. ferrophilus* (Pfe) DNA PolB to allow the 1128 Cren7 domain of *Hyperthermus butylicus* to be fused to its C-terminal.

The following primers introduced the BamHI site to the C-terminal end:
13482_FL_Start*_*(*Nde*I), SEQ ID NO:25 (above)
Pfe_Lower(BamHI) SEQ ID NO:48:
   5'-GAATTCGTCGACTTAGGATCCCTTCTTCCCCTTCGGCTTTAACCA-3'

Pfe DNA PolB(BamHI) was PCR amplified using Phusion DNA polymerase (NEB) and plasmid DNA from an active Pfe DNA PolB clone as template. The Pfe DNA PolB(BamHI) nucleotide had the nucleic acid sequence:

The BamHI recognition sequence is boxed and the start and stop codons capitalised.

Cren7 from Hyperthermus butilicus gDNA was amplified using the following primers:
Hbu_cren7_upper(BamHI) SEQ ID NO:50:
   5'-GAATTCGGATCCGGAACACACATGGCGTGTGAGAAGCCT G-3'
Hbu_cren7_lower(SalI) SEQ ID NO:51:
   5'-GAATTCGTCGACTTAGCTGCAGATTGGGTA-3'

The Hbu_cren7(BamHI) nucleotide had the following nucleic acid sequence (BamHI recognition sequence boxed in lower case, stop codon capitalised and SalI recognition sequence boxed and capitalised):

This was directionally cloned into the Pfe_DNA_PolB between BamHI/SalI restriction sites.

## Claims

1. A polypeptide having thermostable DNA polymerase activity and comprising or consisting of an amino acid sequence with at least 94% identity to *Palaeococcus ferrophilus* DNA polymerase shown in SEQ ID NO:1.

2. The polypeptide according to claim 1, which has a half-life at 95°C of about 0.5-6 h, such as about 1-4 h or about 1 h.

3. The polypeptide according to any preceding claim, in which the polypeptide has 3'-5' exonuclease proofreading activity, or in which the polypeptide lacks 5'-3' exonuclease activity.

4. The polypeptide according to any preceding claim, which is an isolated thermostable DNA polymerase obtainable from *Palaeococcus ferrophilus* and having a molecular weight of about 90,000 Daltons, or a fragment thereof having thermostable DNA polymerase activity.

5. The polypeptide according to any preceding claim comprising the amino acid sequence RQRAIKILANSYYGYYGYAR (SEQ ID NO:35) or a portion thereof comprising "NS".

6. The polypeptide according to any of claims 1-3, comprising or consisting of an amino acid sequence with at least 81% identity to *Palaeococcus ferrophilus* DNA polymerase intein protein shown in SEQ ID NO:2.

7. A polypeptide according to claim 1 having thermostable DNA polymerase activity and comprising the amino acid sequence SEQ ID NO:1.

8. A polypeptide according to any preceding claim, further comprising a Cren7 enhancer domain.

9. A polypeptide according to claim 8 comprising the amino acid sequence SEQ ID NO: 46.

10. A composition comprising the polypeptide of any of claims 1-9.

11. A nucleic acid encoding the polypeptide of any of claims 1-5 or 7, for example having the nucleotide sequence SEQ ID NO:3, or encoding the polypeptide of claim 6, for example having the nucleotide sequence SEQ ID NO:4, or encoding the polypeptide of claim 8 or 9.

12. A vector comprising the nucleic acid of claim 11.

13. A host cell transformed with the vector of claim 12.

14. A kit comprising the polypeptide of any of claims 1-9, and/or the composition of claim 10, and/or the nucleic acid of claim 11, and/or the vector of claim 12, and/or the host cell of claim 13, together with packaging materials therefor.

15. A method of amplifying a sequence of a target nucleic acid using a thermocycling reaction, comprising the steps of:
(1) contacting the target nucleic acid with the polypeptide of any of claims 1-9, and/or the composition of claim 10; and
(2) incubating the target nucleic acid with the polypeptide and/or composition under thermocycling reaction conditions which allow amplification of the target nucleic acid.

## Patentansprüche

1. Polypeptid, das eine thermostabile DNA-Polymerase-Aktivität aufweist und das eine Aminosäuresequenz mit mindestens 94 % Identität mit einer in SEQ ID NO:1 dargestellten *Palaeococcus ferrophilus* DNA-Polymerase umfasst oder aus einer solchen besteht.

2. Polypeptid gemäß Anspruch 1, das eine Halbwertszeit bei 95 °C von etwa 0,5-6 h hat, beispielsweise von etwa 1-4 h oder von etwa 1 h.

3. Polypeptid gemäß einem der vorangehenden Ansprüche, wobei das Polypeptid eine 3'-5' Exonuklease-Proofreading-Aktivität aufweist oder in dem Polypeptid die 5'-3' Exonuklease-Aktivität fehlt.

4. Polypeptid gemäß einem der vorangehenden Ansprüche, das eine isolierte, thermostabile, aus *Palaeococcus ferrophilus* erhältliche DNA-Polymerase ist und ein Molekulargewicht von etwa 90.000 Dalton aufweist, oder ein Fragment davon mit thermostabiler DNA-Polymerase-Aktivität.

5. Polypeptid gemäß einem der vorangehenden Ansprüche, das die Aminosäuresequenz RQRAIKILANSYYGYYGYAR (SEQ ID NO:35) oder einen Teil davon umfasst, der "NS" umfasst.

6. Polypeptid gemäß einem der Ansprüche 1 bis 3, das eine Aminosäuresequenz mit mindestens 81 % Identität mit einem in SEQ ID NO:2 dargestellten *Palaeococcus ferrophilus* DNA-Polymerase-Intein-Protein umfasst oder aus einer solchen besteht.

7. Polypeptid gemäß Anspruch 1, das eine thermostabile DNA-Polymerase-Aktivität aufweist und die Aminosäuresequenz SEQ ID NO:1 umfasst.

8. Polypeptid gemäß einem der vorangehenden Ansprüche, das ferner eine Cren7 Enhancer-Domäne umfasst.

9. Polypeptid gemäß Anspruch 8, das die Aminosäuresequenz SEQ ID NO:46 umfasst.

10. Zusammensetzung, die das Polypeptid nach einem der Ansprüche 1 bis 9 umfasst.

11. Nukleinsäure, die das Polypeptid nach einem der Ansprüche 1 bis 5 oder 7 codiert, beispielsweise die Nukleotidsequenz SEQ ID NO:3 aufweisend, oder die das Polypeptid nach Anspruch 6 codiert, beispielsweise die Nukleotidsequenz SEQ ID NO:4 aufweisend, oder die das Polypeptid nach Anspruch 8 oder 9 codiert.

12. Vektor, der die Nukleinsäure nach Anspruch 11 umfasst.

13. Wirtszelle, die mit dem Vektor nach Anspruch 12 transformiert wurde.

14. Kit, das das Polypeptid nach einem der Ansprüche 1 bis 9 und/oder die Zusammensetzung nach Anspruch 10 und/oder die Nukleinsäure nach Anspruch 11 und/oder den Vektor nach Anspruch 12 und/oder die Wirtszelle nach Anspruch 13 gemeinsam mit den Verpackungsmaterialien hierfür umfasst.

15. Verfahren zur Amplifizierung einer Sequenz einer Zielnukleinsäure unter Verwendung einer Thermocycling-Reaktion, umfassend die Schritte:
(1) Kontaktieren der Zielnukleinsäure mit dem Polypeptid nach einem der Ansprüche 1 bis 9 und/oder der Zusammensetzung nach Anspruch 10; und
(2) Inkubieren der Zielnukleinsäure mit dem Polypeptid und/oder der Zusammensetzung unter Thermocycling-Reaktionsbedingungen, die eine Amplifikation der Zielnukleinsäure ermöglichen.

## Revendications

1. Polypeptide possédant une activité d'ADN polymérase thermostable et comprenant ou consistant en une séquence d'acides aminés identique à au moins 94 % à l'ADN polymérase de *Palaeococcus ferrophilus* représentée par SEQ ID NO : 1.

2. Polypeptide selon la revendication 1, qui a une demi-vie à 95 °C d'environ 0,5 à 6 heures, comme environ 1 à 4 heures ou environ 1 heure.

3. Polypeptide selon l'une quelconque des revendications précédentes, dans lequel le polypeptide possède une activité de relecture d'exonucléase 3'-5', ou dans lequel le polypeptide est dépourvu d'une activité d'exonucléase 5'-3'.

4. Polypeptide selon l'une quelconque des revendications précédentes, qui est une ADN polymérase thermostable isolée pouvant être obtenue à partir de *Palaeococcus ferrophilus* et possédant un poids moléculaire d'environ 90 000 Daltons, ou un fragment de celle-ci possédant une activité d'ADN polymérase thermostable.

5. Polypeptide selon l'une quelconque des revendications précédentes, comprenant la séquence d'acides aminés RQRAIKILANSYYGYYGYAR (SEQ ID NO : 35) ou une partie de celle-ci comprenant « NS ».

6. Polypeptide selon l'une quelconque des revendications 1 à 3, comprenant ou consistant en une séquence d'acides aminés qui est identique à au moins 81 % à la protéine intéine de l'ADN polymérase de *Palaeococcus ferrophilus* représentée par SEQ ID NO : 2.

7. Polypeptide selon la revendication 1, possédant une activité d'ADN polymérase thermostable et comprenant la séquence d'acides aminés de SEQ ID NO : 1.

8. Polypeptide selon l'une quelconque des revendications précédentes, comprenant en outre un domaine amplificateur Cren7.

9. Polypeptide selon la revendication 8, comprenant la séquence d'acides aminés de SEQ ID NO : 46.

10. Composition comprenant le polypeptide selon l'une quelconque des revendications 1 à 9.

11. Acide nucléique codant le polypeptide selon l'une quelconque des revendications 1 à 5 ou 7, possédant par exemple la séquence nucléotidique de SEQ ID NO : 3, ou codant le polypeptide selon la revendication 6, possédant par exemple la séquence nucléotidique de SEQ ID NO : 4, ou codant le polypeptide selon la revendication 8 ou 9.

12. Vecteur comprenant l'acide nucléique de la revendication 11.

13. Cellule hôte transformée avec le vecteur de la revendication 12.

14. Kit comprenant le polypeptide selon l'une quelconque des revendications 1 à 9 et/ou la composition selon la revendication 10 et/ou l'acide nucléique selon la revendication 11 et/ou le vecteur selon la revendication 12 et/ou la cellule hôte selon la revendication 13, conjointement avec des matériaux permettant leur conditionnement.

15. Méthode d'amplification d'une séquence d'un acide nucléique cible par une réaction de thermocyclage, comprenant les étapes suivantes :
(1) le contact de l'acide nucléique cible avec le polypeptide selon l'une quelconque des revendications 1 à 9 et/ou la composition selon la revendication 10 ; et
(2) l'incubation de l'acide nucléique cible avec le polypeptide et/ou la composition dans des conditions réactionnelles de thermocyclage qui permettent l'amplification de l'acide nucléique cible.
